# EUROPEAN PATENT SPECIFICATION

(11) **EP 2 553 445 B1**
(45) Date of publication and mention of the grant of the patent: **30.03.2016**
(21) Application number: 11763422.0
(22) Date of filing: 31.03.2011
(51) Int. Cl.: G01N 33/574, C12Q 1/68

(54) **GENE-BASED PREDICTION OF PSA RECURRENCE FOR CLINICALLY LOCALIZED PROSTATE CANCER PATIENTS**
GENBASIERTE PROGNOSE EINES PSA-WIEDERAUFTRETENS BEI PATIENTEN MIT KLINISCH LOKALISIERTEM PROSTATAKREBS
PRÉDICTION GÉNÉTIQUE DE RÉCIDIVE DU PSA POUR DES PATIENTS DU CANCER DE LA PROSTATE EN MILIEU CLINIQUE

(30) Priority: 02.04.2010 US 320398 P
(43) Date of publication of application: 06.02.2013
(73) Proprietor: Janssen Diagnostics, LLC, Raritan, NJ 08869 (US)
(72) Inventor: TALANTOV, Dimitri, San Diego, CA 92130 (US); JATKOE, Timothy, Bedminster, NJ 07921-1930 (US); ZHANG, Yi, San Diego, CA 92127 (US); WANG, Yixin, Basking Ridge, NJ 07920 (US); PALMA, John, F., Carlsbad, CA 92009 (US)
(74) Representative: Carpmaels & Ransford LLP
(86) International application number: PCT/US2011/030686
(87) International publication number: WO 2011/123615

(56) References cited:
- WO-A1-2008/143896
- WO-A1-2009/143603
- WO-A2-2008/031839
- US-A1- 2003 054 419
- US-A1- 2003 235 816
- "GeneChip Human Genome U95 Set", , 1 January 2003 (2003-01-01), XP055076997, Retrieved from the Internet: URL:http://media.affymetrix.com/support/te chnical/datasheets/hgu95_datasheet.pdf [retrieved on 2013-08-29]
- BASTIAN P J ET AL: "GLOBAL HISTONE MODIFICATIONS AS A PROGNOSTIC MARKER FOR BIOCHEMICAL RECURRENCE IN PATIENTS UNDERGOING RADICAL PROSTATECTOMY", JOURNAL OF UROLOGY, LIPPINCOTT WILLIAMS & WILKINS, BALTIMORE, MD, US, vol. 181, no. 4, 1 April 2009 (2009-04-01) , pages 185-186, XP026008583, ISSN: 0022-5347, DOI: 10.1016/S0022-5347(09)60534-6 [retrieved on 2009-03-18]
- GLINSKY G V ET AL: "Gene expression profiling predicts clinical outcome of prostate cancer", JOURNAL OF CLINICAL INVESTIGATION, AMERICAN SOCIETY FOR CLINICAL INVESTIGATION, US, vol. 113, no. 6, 1 March 2004 (2004-03-01) , pages 913-923, XP002395330, ISSN: 0021-9738, DOI: 10.1172/JCI200420032
- TOHRU NAKAGAWA ET AL: "A Tissue Biomarker Panel Predicting Systemic Progression after PSA Recurrence Post-Definitive Prostate Cancer Therapy", PLOS ONE, vol. 3, no. 5, 28 May 2008 (2008-05-28), page e2318, XP055010915, DOI: 10.1371/journal.pone.0002318
- L. A. MUCCI ET AL: "Testing a Multigene Signature of Prostate Cancer Death in the Swedish Watchful Waiting Cohort", CANCER EPIDEMIOLOGY, BIOMARKERS & PREVENTION, vol. 17, no. 7, 1 July 2008 (2008-07-01), pages 1682-1688, XP055076563, ISSN: 1055-9965, DOI: 10.1158/1055-9965.EPI-08-0044
- S. F. SHARIAT ET AL: "Comparison of Nomograms With Other Methods for Predicting Outcomes in Prostate Cancer: A Critical Analysis of the Literature", CLINICAL CANCER RESEARCH, vol. 14, no. 14, 15 July 2008 (2008-07-15) , pages 4400-4407, XP055076560, ISSN: 1078-0432, DOI: 10.1158/1078-0432.CCR-07-4713
- OSAMU OKAMOTO ET AL: "Dermatopontin Promotes Epidermal Keratinocyte Adhesian via .alpha.3.beta.1 Integrin and a Proteoglycan Receptor", BIOCHEMISTRY, AMERICAN CHEMICAL SOCIETY, US, vol. 49, no. 1, 12 January 2010 (2010-01-12), pages 147-155, XP002632716, ISSN: 0006-2960, DOI: 10.1021/BI901066F [retrieved on 2009-11-24]
- KRISTIANSEN GLEN ET AL: "Expression profiling of microdissected matched prostate cancer samples reveals CD166/MEMD and CD24 as new prognostic markers for patient survival", JOURNAL OF PATHOLOGY, JOHN WILEY & SONS LTD, GB, vol. 205, no. 3, 1 February 2005 (2005-02-01), pages 359-376, XP002556131, ISSN: 0022-3417, DOI: 10.1002/PATH.1676 [retrieved on 2004-11-08]
- OHL ET AL.: 'Gene expression studies in prostate cancer tissue: which reference gene should be selected for normalization?' J MOL MED vol. 83, no. 12, December 2005, pages 1014 - 1024, XP019320313
- TAKEUCHI ET AL.: 'Extracellular matrix dermatopontin modulates prostate cell growth in vivo.' J ENDOCRINOL vol. 190, no. 2, August 2006, pages 351 - 361, XP055099923
- POPOVICI ET AL.: 'Selecting control genes for RT-QPCR using public microarray data.' BMC BIOINFORMATICS, [Online] vol. 10, no. 42, 02 February 2009, pages 1 - 10, XP021047304 Retrieved from the Internet: <URL:http://www.biomedcentral.com/1471-2105 /10/42>
- YU ET AL.: 'Gene expression alterations in prostate cancer predicting tumor aggression and preceding development of malignancy.' J CLIN ONCO vol. 14, no. 22, 15 July 2004, pages 2790 - 2799, XP002551983
- TALANTOV ET AL.: 'Gene based prediction of clinically localized prostate cancer progression after radical prostatectomy.' J UROL vol. 184, no. 4, 17 August 2007, pages 1521 - 1528, XP027275155
- PARIS ET AL.: 'A Group of Genome-Based Biomarkers That Add to a Kattan Nomogram for Predicting Progression in Men with High-Risk Prostate Cancer.' CLIN CAN RES vol. 16, no. 1, 22 December 2009, pages 195 - 202, XP055076497

### SEQUENCE LISTING

<110> Veridex, LLC
<120> GENE-BASED PREDICTION OF PSA RECURRENCE FOR CLINICALLY LOCALIZED PROSTATE CANCER PATIENTS
<130> P060431EP
<150> 61/320398 <151> 2010-04-02
<160> 99
<170> PatentIn version 3.5
<210> 1
   <211> 21
   <212> DNA
   <213> Artificial Sequence
<220>
   <223> Oligonucleotide primer
<400> 1
   gggttggaag gatttcctga a 21
<210> 2
   <211> 23
   <212> DNA
   <213> Artificial Sequence
<220>
   <223> Oligonucleotide primer
<400> 2
   ccctgcactc attttcctta ctg 23
<210> 3
   <211> 22
   <212> DNA
   <213> Artificial Sequence
<220>
   <223> Oligonucleotide Probe
<400> 3
   tagaagacaa acgttagcat ac 22
<210> 4
   <211> 22
   <212> DNA
   <213> Artificial Sequence
<220>
   <223> Oligonucleotide primer
<400> 4
   gcactcaaga gcaagctcag ag 22
<210> 5
   <211> 18
   <212> DNA
   <213> Artificial Sequence
<220>
   <223> Oligonucleotide primer
<400> 5
   tcgtttcctc gcctggtg 18
<210> 6
   <211> 18
   <212> DNA
   <213> Artificial Sequence
<220>
   <223> Oligonucleotide probe
<400> 6
   aggaaacttc gcagtgat 18
<210> 7
   <211> 24
   <212> DNA
   <213> Artificial Sequence
<220>
   <223> Oligonucleotide primer
<400> 7
   agtttaccaa ctgggtgatg tcag 24
<210> 8
   <211> 22
   <212> DNA
   <213> Artificial Sequence
<220>
   <223> Oligonucleotide primer
<400> 8
   ttgatatgcc acgtctctga gg 22
<210> 9
   <211> 17
   <212> DNA
   <213> Artificial Sequence
<220>
   <223> Oligonucleotide probe
<400> 9
   atggcacaga atatcag 17
<210> 10
   <211> 19
   <212> DNA
   <213> Artificial Sequence
<220>
   <223> Oligonucleotide primer
<400> 10
   tgcagtggaa agggatcgc 19
<210> 11
   <211> 20
   <212> DNA
   <213> Artificial Sequence
<220>
   <223> Oligonucleotide primer
<400> 11
   cccagatttg gtatgtggca 20
<210> 12
   <211> 24
   <212> DNA
   <213> Artificial Sequence
<220>
   <223> Oligonucleotide probe
<400> 12
   cataatgtgc cggatgactg aata 24
<210> 13
   <211> 21
   <212> DNA
   <213> Artificial Sequence
<220>
   <223> Oligonucleotide primer
<400> 13
   gcactcaaga gcaagctcag a 21
<210> 14
   <211> 22
   <212> DNA
   <213> Artificial Sequence
<220>
   <223> Oligonucleotide primer
<400> 14
   tagaaggaac gaaagaggtc tc 22
<210> 15
   <211> 22
   <212> DNA
   <213> Artificial Sequence
<220>
   <223> Oligonucleotide probe
<400> 15
   ccacaggaaa cttcgcagtg at 22
<210> 16
   <211> 21
   <212> DNA
   <213> Artificial Sequence
<220>
   <223> Oligonucleotide primer
<400> 16
   gggatagtga agtttaccaa c 21
<210> 17
   <211> 20
   <212> DNA
   <213> Artificial Sequence
<220>
   <223> Oligonucleotide primer
<400> 17
   ttgatatgcc acgtctctga 20
<210> 18
   <211> 24
   <212> DNA
   <213> Artificial Sequence
<220>
   <223> Oligonucleotide probe
<400> 18
   cagtcaaaag acaacatggc acag 24
<210> 19
   <211> 16
   <212> DNA
   <213> Artificial Sequence
<220>
   <223> Oligonucleotide primer
<400> 19
   ttcgcaagct ggctga 16
<210> 20
   <211> 18
   <212> DNA
   <213> Artificial Sequence
<220>
   <223> Oligonucleotide primer
<400> 20
   catgagcagg gaggtgaa 18
<210> 21
   <211> 24
   <212> DNA
   <213> Artificial Sequence
<220>
   <223> Oligonucleotide probe
<400> 21
   agctttggtg ggggaactgg ttct 24
<210> 22
   <211> 19
   <212> DNA
   <213> Artificial Sequence
<220>
   <223> Oligonucleotide primer
<400> 22
   caagtgtcag tctggtgca 19
<210> 23
   <211> 22
   <212> DNA
   <213> Artificial Sequence
<220>
   <223> Oligonucleotide primer
<400> 23
   gttgtttcaa agtgtccata ac 22
<210> 24
   <211> 22
   <212> DNA
   <213> Artificial Sequence
<220>
   <223> Oligonucleotide probe
<400> 24
   ctaggaatga gtcgccaccc ac 22
<210> 25
   <211> 19
   <212> DNA
   <213> Artificial Sequence
<220>
   <223> Oligonucleotide primer
<400> 25
   agccttcctt cctgggtat 19
<210> 26
   <211> 19
   <212> DNA
   <213> Artificial Sequence
<220>
   <223> Oligonucleotide primer
<400> 26
   tgatggagtt gaaggtggt 19
<210> 27
   <211> 20
   <212> DNA
   <213> Artificial Sequence
<220>
   <223> Oligonucleotide probe
<400> 27
   gaatcttgcg gcatccacga 20
<210> 28
   <211> 13
   <212> DNA
   <213> Artificial Sequence
<220>
   <223> ACTG1 Oligonucleotide probe
<400> 28
   ttgcggcatc cac 13
<210> 29
   <211> 21
   <212> DNA
   <213> Artificial Sequence
<220>
   <223> ACTG1 Oligonucleotide primer
<400> 29
   cagccttcct tcctgggtat g 21
<210> 30
   <211> 23
   <212> DNA
   <213> Artificial Sequence
<220>
   <223> ACTG1 Oligonucleotide primer
<400> 30
   catgatggag ttgaaggtgg tct 23
<210> 31
   <211> 19
   <212> DNA
   <213> Artificial Sequence
<220>
   <223> ACTG2 Oligonucleotide probe
<400> 31
   catgagacaa cctacaatt 19
<210> 32
   <211> 20
   <212> DNA
   <213> Artificial Sequence
<220>
   <223> ACTG2 Oligonucleotide primer
<400> 32
   tttattggca tggagtccgc 20
<210> 33
   <211> 24
   <212> DNA
   <213> Artificial Sequence
<220>
   <223> ACTG2 Oligonucleotide primer
<400> 33
   ccttacggat gtcaatgtca cact 24
<210> 34
   <211> 21
   <212> DNA
   <213> Artificial Sequence
<220>
   <223> ALAS1 Oligonucleotide probe
<400> 34
   cagtatgatc gtttctttga g 21
<210> 35
   <211> 26
   <212> DNA
   <213> Artificial Sequence
<220>
   <223> ALAS1 Oligonucleotide primer
<400> 35
   ataacttgcc aaaatctgtt tccact 26
<210> 36
   <211> 25
   <212> DNA
   <213> Artificial Sequence
<220>
   <223> ALAS1 Oligonucleotide primer
<400> 36
   aaactcgata ggtgtggtca ttctt 25
<210> 37
   <211> 18
   <212> DNA
   <213> Artificial Sequence
<220>
   <223> CALD1 Oligonucleotide probe
<400> 37
   atgcctgatg acctataa 18
<210> 38
   <211> 28
   <212> DNA
   <213> Artificial Sequence
<220>
   <223> CALD1 Oligonucleotide primer
<400> 38
   catggcagat aggtatcaat atgttttc 28
<210> 39
   <211> 30
   <212> DNA
   <213> Artificial Sequence
<220>
   <223> CALD1 Oligonucleotide primer
<400> 39
   tcaactcctt ctaacagttc taatctctct 30
<210> 40
   <211> 19
   <212> DNA
   <213> Artificial Sequence
<220>
   <223> CBX3 Oligonucleotide probe
<400> 40
   atttgccaga ggtcttgat 19
<210> 41
   <211> 23
   <212> DNA
   <213> Artificial Sequence
<220>
   <223> CBX3 Oligonucleotide primer
<400> 41
   aaagagatgc tgctgacaaa cca 23
<210> 42
   <211> 23
   <212> DNA
   <213> Artificial Sequence
<220>
   <223> CBX3 Oligonucleotide primer
<400> 42
   catcaattct ccactgctgt ctg 23
<210> 43
   <211> 17
   <212> DNA
   <213> Artificial Sequence
<220>
   <223> DCHS1 Oligonucleotide probe
<400> 43
   tgaacagctc aacaggg 17
<210> 44
   <211> 17
   <212> DNA
   <213> Artificial Sequence
<220>
   <223> DCHS1 Oligonucleotide primer
<400> 44
   gccgtgaggc atttgca 17
<210> 45
   <211> 17
   <212> DNA
   <213> Artificial Sequence
<220>
   <223> DCHS1 Oligonucleotide primer
<400> 45
   cactcgcgca cgcaact 17
<210> 46
   <211> 16
   <212> DNA
   <213> Artificial Sequence
<220>
   <223> DKK3 Oligonucleotide probe
<400> 46
   cagactggac aaatgg 16
<210> 47
   <211> 25
   <212> DNA
   <213> Artificial Sequence
<220>
   <223> DKK3 Oligonucleotide primer
<400> 47
   cgagaaattc acaagataac caaca 25
<210> 48
   <211> 20
   <212> DNA
   <213> Artificial Sequence
<220>
   <223> DKK3 Oligonucleotide primer
<400> 48
   ctgccttctt cgtctcccac 20
<210> 49
   <211> 13
   <212> DNA
   <213> Artificial Sequence
<220>
   <223> FLNA Oligonucleotide probe
<400> 49
   atggcccaag gac 13
<210> 50
   <211> 21
   <212> DNA
   <213> Artificial Sequence
<220>
   <223> FLNA Oligonucleotide primer
<400> 50
   cagcaaagca ggcaacaaca t 21
<210> 51
   <211> 20
   <212> DNA
   <213> Artificial Sequence
<220>
   <223> FLNA Oligonucleotide primer
<400> 51
   cgtgcttcac caggatctcc 20
<210> 52
   <211> 20
   <212> DNA
   <213> Artificial Sequence
<220>
   <223> FLNC Oligonucleotide probe
<400> 52
   caaccccaga gttttaagga 20
<210> 53
   <211> 21
   <212> DNA
   <213> Artificial Sequence
<220>
   <223> FLNC Oligonucleotide primer
<400> 53
   ggtctggtct ctctggtggc t 21
<210> 54
   <211> 24
   <212> DNA
   <213> Artificial Sequence
<220>
   <223> FLNC Oligonucleotide primer
<400> 54
   ttctctgatt gtgctttcct ttcc 24
<210> 55
   <211> 23
   <212> DNA
   <213> Artificial Sequence
<220>
   <223> GAS1 Oligonucleotide probe
<400> 55
   tatagaatcc atttgtcatc agg 23
<210> 56
   <211> 26
   <212> DNA
   <213> Artificial Sequence
<220>
   <223> GAS1 Oligonucleotide primer
<400> 56
   actcacatcc atattacacc tttccc 26
<210> 57
   <211> 29
   <212> DNA
   <213> Artificial Sequence
<220>
   <223> GAS1 Oligonucleotide primer
<400> 57
   taaatatagc acacttcaca atggactgt 29
<210> 58
   <211> 16
   <212> DNA
   <213> Artificial Sequence
<220>
   <223> GSN Oligonucleotide probe
<400> 58
   ccgagttcct caaggc 16
<210> 59
   <211> 16
   <212> DNA
   <213> Artificial Sequence
<220>
   <223> GSN Oligonucleotide primer
<400> 59
   gcggcccaac agcatg 16
<210> 60
   <211> 17
   <212> DNA
   <213> Artificial Sequence
<220>
   <223> GSN Oligonucleotide primer
<400> 60
   tgcaggccag gctcctt 17
<210> 61
   <211> 19
   <212> DNA
   <213> Artificial Sequence
<220>
   <223> HIST1H3D Oligonucleotide probe
<400> 61
   aagttcgcaa tggctcgta 19
<210> 62
   <211> 21
   <212> DNA
   <213> Artificial Sequence
<220>
   <223> HIST1H3D Oligonucleotide primer
<400> 62
   caaggccaag gcaggtttta g 21
<210> 63
   <211> 18
   <212> DNA
   <213> Artificial Sequence
<220>
   <223> HIST1H3D Oligonucleotide primer
<400> 63
   cacccgtgga cttgcgag 18
<210> 64
   <211> 15
   <212> DNA
   <213> Artificial Sequence
<220>
   <223> LIMS2 Oligonucleotide probe
<400> 64
   tccacaccca caagc 15
<210> 65
   <211> 21
   <212> DNA
   <213> Artificial Sequence
<220>
   <223> LIMS2 Oligonucleotide primer
<400> 65
   cacactgagc cagcaagtcc t 21
<210> 66
   <211> 19
   <212> DNA
   <213> Artificial Sequence
<220>
   <223> LIMS2 Oligonucleotide primer
<400> 66
   ttccgaagga tggaggtgg 19
<210> 67
   <211> 19
   <212> DNA
   <213> Artificial Sequence
<220>
   <223> LMOD1 Oligonucleotide probe
<400> 67
   ctgaactgtg agtcctgat 19
<210> 68
   <211> 18
   <212> DNA
   <213> Artificial Sequence
<220>
   <223> LMOD1 Oligonucleotide primer
<400> 68
   gctgtgcccc acctgttg 18
<210> 69
   <211> 20
   <212> DNA
   <213> Artificial Sequence
<220>
   <223> LMOD1 Oligonucleotide primer
<400> 69
   tagagtcctc cagggagccc 20
<210> 70
   <211> 18
   <212> DNA
   <213> Artificial Sequence
<220>
   <223> MT1X Oligonucleotide probe
<400> 70
   ctcgaaatgg accccaac 18
<210> 71
   <211> 21
   <212> DNA
   <213> Artificial Sequence
<220>
   <223> MT1X Oligonucleotide primer
<400> 71
   gatcgggaac tcctgcttct c 21
<210> 72
   <211> 18
   <212> DNA
   <213> Artificial Sequence
<220>
   <223> MT1X Oligonucleotide primer
<400> 72
   caggagccaa caggcgag 18
<210> 73
   <211> 20
   <212> DNA
   <213> Artificial Sequence
<220>
   <223> MYLK Oligonucleotide probe
<400> 73
   tctgaagaag atgtgtccca 20
<210> 74
   <211> 16
   <212> DNA
   <213> Artificial Sequence
<220>
   <223> MYLK Oligonucleotide primer
<400> 74
   ccagcccgct caatgc 16
<210> 75
   <211> 21
   <212> DNA
   <213> Artificial Sequence
<220>
   <223> MYLK Oligonucleotide primer
<400> 75
   ctcagcaaca gcctcaagga a 21
<210> 76
   <211> 20
   <212> DNA
   <213> Artificial Sequence
<220>
   <223> PDLIM3 Oligonucleotide probe
<400> 76
   gaagatcaca ccttttaatg 20
<210> 77
   <211> 23
   <212> DNA
   <213> Artificial Sequence
<220>
   <223> PDLIM3 Oligonucleotide primer
<400> 77
   ggataatggc aagccactca taa 23
<210> 78
   <211> 25
   <212> DNA
   <213> Artificial Sequence
<220>
   <223> PDLIM3 Oligonucleotide primer
<400> 78
   tctgtttctc tccttctctc ttcca 25
<210> 79
   <211> 19
   <212> DNA
   <213> Artificial Sequence
<220>
   <223> PDLIM7 Oligonucleotide probe
<400> 79
   ctgaagatga cctggcacg 19
<210> 80
   <211> 25
   <212> DNA
   <213> Artificial Sequence
<220>
   <223> PDLIM7 Oligonucleotide primer
<400> 80
   gaagaagatt acaggcgaga tcatg 25
<210> 81
   <211> 20
   <212> DNA
   <213> Artificial Sequence
<220>
   <223> PDLIM7 Oligonucleotide primer
<400> 81
   caggcagcac aggtaaagca 20
<210> 82
   <211> 22
   <212> DNA
   <213> Artificial Sequence
<220>
   <223> RASL12 Oligonucleotide probe
<400> 82
   cttcccgacc cacaggccag ct 22
<210> 83
   <211> 22
   <212> DNA
   <213> Artificial Sequence
<220>
   <223> RASL12 Oligonucleotide primer
<400> 83
   accacatgct tgcagtccta ca 22
<210> 84
   <211> 20
   <212> DNA
   <213> Artificial Sequence
<220>
   <223> RASL12 Oligonucleotide primer
<400> 84
   agtggcctgg agcaaaagtg 20
<210> 85
   <211> 21
   <212> DNA
   <213> Artificial Sequence
<220>
   <223> SH3BGRL Oligonucleotide probe
<400> 85
   ctagcaaaga gattagactt t 21
<210> 86
   <211> 22
   <212> DNA
   <213> Artificial Sequence
<220>
   <223> SH3BGRL Oligonucleotide primer
<400> 86
   catgaagtgg gatgccaagt aa 22
<210> 87
   <211> 25
   <212> DNA
   <213> Artificial Sequence
<220>
   <223> SH3BGRL Oligonucleotide primer
<400> 87
   gatcgccaac ctgttttata agagt 25
<210> 88
   <211> 18
   <212> DNA
   <213> Artificial Sequence
<220>
   <223> SMTN Oligonucleotide probe
<400> 88
   ttcacctatg tgcagtcg 18
<210> 89
   <211> 19
   <212> DNA
   <213> Artificial Sequence
<220>
   <223> SMTN Oligonucleotide primer
<400> 89
   gcaagaagcc tgaccccaa 19
<210> 90
   <211> 16
   <212> DNA
   <213> Artificial Sequence
<220>
   <223> SMTN Oligonucleotide primer
<400> 90
   tcgtggcgtc gcaggt 16
<210> 91
   <211> 24
   <212> DNA
   <213> Artificial Sequence
<220>
   <223> SORBS1 Oligonucleotide probe
<400> 91
   ctttaatggt gatacacaag taga 24
<210> 92
   <211> 22
   <212> DNA
   <213> Artificial Sequence
<220>
   <223> SORBS1 Oligonucleotide primer
<400> 92
   ccagtgcagg ttttggaata tg 22
<210> 93
   <211> 23
   <212> DNA
   <213> Artificial Sequence
<220>
   <223> SORBS1 Oligonucleotide primer
<400> 93
   tgatcctctc accctttctg aag 23
<210> 94
   <211> 15
   <212> DNA
   <213> Artificial Sequence
<220>
   <223> TNS1 Oligonucleotide probe
<400> 94
   cacggcatcc ccaac 15
<210> 95
   <211> 20
   <212> DNA
   <213> Artificial Sequence
<220>
   <223> TNS1 Oligonucleotide primer
<400> 95
   aagcccttgt ttctgcacca 20
<210> 96
   <211> 22
   <212> DNA
   <213> Artificial Sequence
<220>
   <223> TNS1 Oligonucleotide primer
<400> 96
   gccgacatcc tcctttagac tc 22
<210> 97
   <211> 17
   <212> DNA
   <213> Artificial Sequence
<220>
   <223> TUBA Oligonucleotide probe
<400> 97
   cgggctgtgt ttgtaga 17
<210> 98
   <211> 25
   <212> DNA
   <213> Artificial Sequence
<220>
   <223> TUBA Oligonucleotide primer
<400> 98
   gactccttca acaccttctt cagtg 25
<210> 99
   <211> 23
   <212> DNA
   <213> Artificial Sequence
<220>
   <223> TUBA Oligonucleotide primer
<400> 99
   tgcgaacttc atcaatgact gtg 23

## Description

### Background

Prior to undergoing radical prostatectomy or other aggressive treatments for prostate cancer, it is useful to know with as much accuracy as possible whether the procedure is likely to be curative. Typically, a physician may provide responses to a patient's request for prognostic information by declining to make specific predictions, or providing overall averages, or providing a subjective assessment or assigning the patient to a defined risk group (such as a high or low risk group) based on a model. Providing the most accurate assessment is almost always the proper response. To provide the best possible assessment, since the pathologic stage of cancer correlates with the probability of recurrence after surgery or treatment, many efforts have been made to predict the final pathologic stage or outcome in prostate cancer patients. To this end, many nomograms, algorithms and markers have been employed with the Kattan Nomogram being the most successful. Preferably, the concordance index is used to select a particular strategy from the considered models/nomograms/algorithms and the like. The Kattan nomogram is described in US5993388 and US6409664.

An aggressive therapy for the treatment of clinically localized prostate cancer is radical prostatectomy. Unfortunately, many men treated with radical prostatectomy later experience progression of their disease. Starting with an increase in serum PSA, which indicates recurrence, the cancer returns in many men months or years following surgery. Early identification, prior to detectable PSA, of men likely to ultimately experience recurrence would be useful in considering additional treatments while preserving quality of life. Further, accurate estimation of a likelihood of recurrence will also be useful in clinical trials to identify candidates for control groups or for an investigational treatment of interest.

Increased accuracy in the classification of newly diagnosed clinically localized prostate cancers is needed if treatment is to be better tailored to this subgroup of patients. As in other cancers, a number of molecular markers and gene signatures of phenotype and prognosis have been developed recently for prostate cancer. These have provided some significant insights into the existence of distinct classes of aggressive prostate cancer and a number of potential candidate gene markers. A clinically viable test that incorporates the expression values of a small number of gene markers is useful either standing alone or in conjunction with other tools such as the Kattan post-operative nomogram to assess risk of PSA recurrence is desirable.

Roupret et al. (2009) Journal of Urology 181(4):186-187 discloses a comparison of pre- and postoperative plasma levels of circulating prostate derived cells in patients with localized prostate cancer allegedly improves prediction of recurrence after radical prostatectomy.

Glinsky et al. (2004) Journal of Clinical Investigation 113(6):913-923 discloses gene expression profiling for allegedly predicting the clinical outcome of prostate cancer.

Nakagawa et al. (2008) PLoS One 3(5):e2318 discloses a tissue biomarker panel for allegedly predicting systemic progression after PSA recurrence post-definitive prostate cancer therapy.

Mucci et al. (2008) Cancer Epidemiology, Biomarkers & Prevention 17(7):1682-1688 discloses an alleged multigene signature of prostate cancer death.

WO 2009/143603 discloses a system for alleged expression-based discrimination of distinct clinical disease states in prostate cancer.

WO 2008/143896 allegedly discloses methylation markers for prostate cancer.

### Summary

The invention provides a method for distinguishing between subjects in a high recurrence risk category of prostate cancer and subjects in a low recurrence risk category of prostate cancer, comprising:
(i) measuring an expression level of at least two diagnostic markers selected from the set of markers, except for Histone 1 H3d, presented in TABLE 2, with each expression level measured relative to an average of expression levels of two or more control markers and
(ii) combining the expression level of the at least two diagnostic markers with
a predictive probability based on a Kattan nomogram, to assign to subjects one of the high recurrence risk category of prostate cancer and the low recurrence risk category of prostate cancer.

The invention further provides a method of predicting the recurrence of prostate cancer comprising:
determining an expression level of DPT relative to a standard;
determining expression level of at least one additional markers from TABLE 2; and
transforming the expression level of DPT and the expression level of at least one additional markers, except for Histone 1 H3d, from TABLE 2 into a score corresponding to a probability of recurrence of prostate cancer.

The invention further provides a kit for detecting an expression level of at least DPT, the kit comprising reagents and a first probe set comprising a first probe specifically recognizing DPT and a second probe specifically recognizing a second Marker, except for Histone 1 H3d, selected from TABLE 2, wherein the kit comprises reagents necessary for RT PCR.

In one aspect of the disclosure, a gene expression based assay is used to provide a prognosis of the recurrence of prostate cancer. The gene expression based assay is preferably based on a small number of genes. A three gene profile is preferred. In a preferred embodiment, the expression level of the three genes MYH11, SSBP1, and DPT provides prognostic information about the likelihood of prostate cancer recurrence. In alternative embodiments, the expression level of genes such as Filamin C, gamma, RAS like family 12 and Filamin A may be used. Other possible marker combinations include (i) Growth Arrest Specific 1, Smoothelin, Leiomodin 1 and Histone 1 H3d, and (ii) Sorbin and SH3 domain containing 1, PDZ and LIM domain 7, LIM and senescent cell-antigen like domains 2. Additional useful combinations of expression level based markers may be readily selected from Table 2.

In another aspect of the disclosure, a gene expression level based assay is used in conjunction with a clinical tool such as a nomogram that is based on multiple clinical indicators of prostate cancer prognosis.

In a preferred embodiment of the disclosure, a measurement of the expression of three genes and three control genes is determined. Preferably, the gene measurements are measured relative to the average of three control genes. These gene measurements along with the predictive probability from the Kattan nomogram are incorporated into a statistical model to generate a score reflecting the probability of recurrence or the risk of recurrence. This information can also be used to determine the likelihood of PSA levels rising following radical prostatectomy. This probability of recurrence can help patients make personalized decisions about their choice in therapy.

More specifically disclosed is a classifier for distinguishing between subjects in a high recurrence risk category of prostate cancer and subjects in a low recurrence risk category of prostate cancer. The preferred classifier comprises a classification procedure including measuring an expression level of at least two diagnostic markers selected from TABLE 2 with each expression level measured relative to an average of the expression levels of two or more control markers. The expression level of the at least two diagnostic markers is then combined with a predictive probability based on a Kattan nomogram to generate the classifier, which may be also be in the form of a nomogram. Herein the Kattan nomogram is a composite measure, described, for instance in US Patent 6,409,664 and 5,993,388.

The Kattan nomogram is typically based on two or more members of a group consisting of PSA value prior to surgery (and prior to hormone therapy, if received), PSA at the time of prostatectomy, primary Gleason at surgery, secondary Gleason at surgery, prostatectomy Gleason Sum, the year of prostatectomy, the months disease free, whether or not surgical margins were positive, whether or not cancer was found in seminal vesicles, whether or not there was extra-capsular extension, whether or not cancer was found in lymph nodes (if any were removed), pre-radiotherapy PSA, radiation dose (if applicable), whether surgical margins were positive or negative (if applicable), whether there was seminal vesicle involvement (if applicable), whether there was lymph node involvement (if applicable), whether there was extra capsular involvement (if applicable), whether or not neo-adjuvant hormones were prescribed, whether or not neo-adjuvant radiation was prescribed.

The Kattan nomogram may also be a composite measure based on two or more members of a group consisting of pretreatment PSA level, combined Gleason grade, specimen Gleason sum, clinical stage, surgical margin status, prostatic capsular invasion maximum cancer length in a core, total length of cancer in the biopsy cores, percent of cores positive level, extraprostatic extension, level of extraprostatic extension, apoptotic index, percent of cancer in one or more cores, percent of high grade cancer in one or more cores, total tumor volume, zone of location of the cancer, presence of seminal vesicle invasion, type of seminal vesicle invasion, p53, Ki-67, p27, DNA ploidy status, lymph node status, and lymphovascular invasion.

A preferred classifier for distinguishing between subjects in a high recurrence risk category of prostate cancer and subjects in a low recurrence risk category of prostate cancer requires measuring the expression levels of diagnostic markers MYH11, SSBP1 and DPT.

Preferred control markers are TUBA, ALAS1 and ACTG1. Alternative preferred control markers are selected for relatively steady expression levels as detected by RT-PCR.

Another preferred classifier for distinguishing between subjects in a high recurrence risk category of prostate cancer and subjects in a low recurrence risk category of prostate cancer requires measuring the expression levels of at least one diagnostic marker selected from TABLE 2 in addition to MYH11, SSBP1 and DPT.

In another preferred embodiment two or more diagnostic markers are selected from the set of markers presented in TABLE 2. More specific sets of diagnostic markers include

Also disclosed is a method of predicting the recurrence of prostate cancer. The method includes the steps of (i) determining an expression level of DPT relative to a standard; (ii) determining expression level of at least one additional markers from TABLE 2; and (iii) transforming the expression level of DPT and the expression level of at least one additional markers from TABLE 2 into a score corresponding to a probability of recurrence of prostate cancer. The method may alternatively, to the step of transforming the expression level of DPT and the expression level of at least one additional markers from TABLE 2, have a step of combining the expression level of DPT and the expression level of at least one additional markers from TABLE 2 with at least one additional indicator of prostate cancer recurrence to determine a composite score. Such a composite score reflects a likelihood of recurrence of prostate cancer.

Preferably, the expression level of DPT and the expression level of at least one additional markers from TABLE 2 is determined prior to at least one treatment selected from the group consisting of prostectomy, hormone therapy, single agent chemotherapy, two agent chemotherapy and treatment with a farnesyl transferase inhibitor.

This disclosure also covers, without limitation, a kit for detecting an expression level of at least DPT, the kit comprising reagents and a first probe set comprising a first probe specifically recognizing DPT and a second probe specifically recognizing a second Marker selected from TABLE 2. The kit may include a device for converting the expression level of DPT and the expression level of at least one additional markers from TABLE 2 into an indicator of a likelihood of recurrence of prostate cancer. The device may be in the form of a nomogram. More specifically, a preferred kit is implemented as one or more members of the group consisting of a mechanical device, a graphical representation, and software instructions to implement a user interface for providing a representation of the indicator of the likelihood of recurrence of prostate cancer. In yet a further aspect of the disclosure a kit is provided containing reagents for conducting a measurement of three genes and three control genes from a prostate tumor. Instructions (optionally as computer code) are provided to enable the gene measurements to be normalized to the average of three control genes. These measures along with the predictive probability from a nomogram are incorporated into a statistical model that generates a probability of recurrence.

### Description of the Figures

Figure 1A shows a comparison of the classifier disclosed herein with the nomogram using the c-index based on results from an independent test series of 157 patients. The c-index for the classifier was apparently higher than the c-index for the nomogram (0.77 vs. 0.67).
Figure 1B shows the correspondence between the 5-year predictive estimates on the test set and the actual probabilities of recurrence. The classifier demonstrated a good calibration across the spectrum of predictions for the test set as compared to an ideal predictor, while the 5-year nomogram displayed less accuracy in detecting the more aggressive cases.
Figure 2A shows a Kaplan-Meier analysis for PSA recurrence-free probability to illustrate the difference in time to PSA recurrence for the predicted low- and high- risk groups (HR 6.85, 95% CI = 3.77 to 12.43, P <.001,). At 5 years, the absolute difference in PSA recurrence between the two groups was 58% (75% vs. 17%).
Figure 2B shows the classifier, used in Figure 2A, applied to patients with Gleason score of 6 or 7.
Figure 2C shows the classifier, used in Figure 2A, applied to patients exhibiting pathological stage pT2 or pT3a.
Figure 2D shows the classifier, used in Figure 2A, applied to patients with pre-operative PSA concentration ≤10 ng/mL or 10<PSA≤20 ng/mL.
Figure 2E shows the classifier, used in Figure 2A, applied to patients with positive or negative surgical margins.
Figure 3 shows the application of the cut-off based on each model's highest accuracy as applied these the test set.

### Detailed Description

Nomograms are widely used to predict prostate cancer recurrence. The most widely used nomogram is the Kattan nomogram described in US Patent 6,409,664 and 5,993,388. For those who have not received surgical treatment for their prostate cancer, these nomograms incorporate the following information: most recent PSA (prostate specific antigen) value, primary and secondary Gleason grade, physician's assessment of clinical stage (using the 1992 or 1997 UICC system), radiation therapy dose that is recommended (if applicable), the number of positive cores found during biopsy, the number of negative cores found during biopsy, whether or not neo-adjuvant hormones had been prescribed, and whether or not neo-adjuvant radiation had been prescribed. For those for whom surgery was performed, the factors include: PSA value prior to surgery (and prior to hormone therapy, if received), PSA at the time of prostatectomy, primary Gleason at surgery, secondary Gleason at surgery, prostatectomy Gleason Sum, the year of prostatectomy, the months disease free, whether or not surgical margins were positive, whether or not cancer was found in seminal vesicles, whether or not there was extra-capsular extension, whether or not cancer was found in lymph nodes (if any were removed), pre-radiotherapy PSA, radiation dose (if applicable), whether surgical margins were positive or negative (if applicable), whether there was seminal vesicle involvement (if applicable), whether there was lymph node involvement (if applicable), whether there was extra capsular involvement (if applicable), whether or not neo-adjuvant hormones were prescribed, whether or not neo-adjuvant radiation was prescribed. This information is combined in a spreadsheet, for example, and a simple statistical treatment provides an analysis used to determine prognosis.

The methods and kits of this invention are most preferably used in conjunction with these nomograms and the information they provide. The method and kits of the invention also involve the detection of a group of genes. Preferably, the expression level one of the genes in this group is measured relative to at least one of the control markers. More preferably the expression levels of three genes and three controls are measured. The most preferred genes are MYH11, SSBP1, and DPT. The preferred controls are ALAS1, TUBA, and ACTG1. Higher expression of the genes MYH11 or DPT indicates a stronger likelihood of remaining free of prostate cancer recurrence than if such over-expression is not seen as described in the examples. A higher expression level of SSBP1 indicates a stronger likelihood of recurrence. The nucleic acid sequences that correspond to the genes whose expression level is measured as well as the sequences used to measure such expression levels are referred to in this specification as Markers. Other sequences of interest include genes useful as assay controls such as ALAS 1, TUBA, and ACTG 1. Markers are detected with any of the methods used to detect gene expression; preferably these are amplification based methods such as PCR, its variants, and alternative methods. Most preferably, RTPCR is used.

Nucleic acid probes or reporters specific for certain Markers are preferably used to detect the expression of the Marker gene in tumor tissue. Other biological fluids or tissues can be used including prostate tissue, urine, urethral washings, blood and blood components such as serum, ejaculate, and other samples from which prostate proteins could be expected. Any specimen containing a detectable amount of the relevant polynucleotide can be used.

One disclosed method includes contacting a target cell containing a Marker with a reagent that binds to the nucleic acid. The target cell component is a nucleic acid such as RNA. The reagents preferably include probes and primers such to amplify and detect the target sequence. For example, the reagents can include priming sequences combined with or bonded to their own reporter segments such as those referred to as Scorpion reagents or Scorpion reporters and described in US Patents 6,326,145 and 6,270,967 to Whitcombe et. al. Though they are not the same, the terms "primers" and "priming sequences" may be used in this specification to refer to molecules or portions of molecules that prime the amplification of nucleic acid sequences.

Preferred primers are capable of initiating synthesis of a primer extension product, which is substantially complementary to a polymorphic locus strand. The primers and/or probes may be prepared using any suitable method including automated methods. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization.

Preferred primers are most preferably eight or more deoxyribonucleotides or ribonucleotides. The exact length of primer will depend on factors such as temperature, buffer, and nucleotide composition. The oligonucleotide primers most preferably contain about 12-20 nucleotides although they may contain more or fewer nucleotides.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 108:1, primer: template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The agent for polymerization may be any compound or system that will function to accomplish the synthesis of primer extension products, preferably enzymes. Suitable enzymes for this purpose include, for example, E. coli DNA polymerase 1, Klenow fragment of E. coli DNA polymerase I, T4 DNA polymerase, other available DNA polymerases, polymerase mutants, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

In another aspect of the invention an expression ratio is used. Establishing a ratio between the amount of amplified Marker attained and the amount of amplified reference Marker or control Marker region amplified can do this. This can be done using quantitative real-time PCR. Ratios can be plugged into a statistical model to determine a likelihood of prostate cancer recurrence.

The kits of the disclosure can be configured with a variety of components, preferably such that they all contain at least one primer or probe or a detection molecule (e.g., Scorpion reporter). In one embodiment, the kit includes reagents for amplifying and detecting Marker segments. Optionally, the kit includes sample preparation reagents and /or articles (e.g., tubes) to extract nucleic acids from samples.

In a preferred kit, reagents necessary for RTPCR are included such as, a corresponding PCR primer set, a thermostable polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to doublestranded DNA such as ethidium bromide can also be used. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like. Optionally, the kits include nucleic acid extraction reagents and materials.

In preferred kit of the disclosure, instructions to conduct the assay on patients with prostate samples are provided. It is most preferred that an article encoded with computer instructions for preparing a prediction from a Cox Proportional Hazard analysis or other statistical comparator is provided. The instructions are loaded into a computer such as a general purpose computer such that when the values of the gene analysis and, optionally, the Kattan parameters are input into the program, the computer provides as output the likelihood of recurrence (high versus low or a numerical indicator).

In a preferred kit of the disclosure, mechanical implementations of a nomogram may be also provided. Such implementations may use paper based components or moving mechanical parts to allow consideration of the individual variables used by the nomogram.

### Examples.

### EXAMPLE 1

**Purpose:** Accurate estimates of the risk of recurrence are needed for the optimal management of patients with clinically localized prostate cancer. A nomogram and novel molecular predictors were combined into a new prognostic model of prostate specific antigen (PSA) recurrence.

This study was designed to identify genes that correlate with PSA recurrence in patients with clinically localized prostate cancer with the goal of developing an accurate predictive classifier that can be readily applied in current routine clinical practice for management of patients with organ-confined disease. Here we report the development of a clinically viable test that incorporates the expression values of three novel gene markers, measurable by RTPCR, and the Kattan post-operative nomogram (11), a widely used tool in the clinical management of prostate cancer, to assess risk of PSA recurrence. Finally, we show that this new classifier provides improved accuracy compared with the Kattan nomogram for predicting biochemical recurrence in this lower risk patient population.

**Materials and Methods:** Gene expression profiles from formalin-fixed, paraffin-embedded (FFPE) localized prostate cancer tissues were analyzed to identify genes associated with PSA recurrence. The profiles of the identified markers were reproduced by reverse-transcriptase-polymerase-chain-reaction (RTPCR). The RTPCR profiles from three of these genes, along with the output from the Kattan post-operative nomogram, were used to produce a predictive model of PSA recurrence.

**Results:** After variable selection, a model of PSA recurrence was built that combined expression values of three genes and the post-operative nomogram. The 3-gene plus nomogram model predicted 5-year PSA recurrence with a concordance index (c-index) of 0.77 in a validation set compared to a c-index of 0.67 for the nomogram. This model identified a subgroup of patients that were at high risk for recurrence which were not identified by the nomogram.

**Conclusions:** This new gene-based classifier has superior predictive power when compared against the 5-year nomogram to assess risk of PSA recurrence in patients with organ-confined prostate cancer. This classifier should provide a more accurate stratification of patients into high and low risk groups for treatment decisions and adjuvant clinical trials.

### MATERIALS AND METHODS

### Patients and tumor samples

Patient information was obtained from the St. Vincent's Campus Prostate Cancer Group (SVCPCG) database (Human Research Ethics Committee Approval H00/088). From January 1990 to December 2001, 960 patients were treated for prostate cancer with radical prostatectomy (RP) with no preoperative therapy at St Vincent's Hospital, Sydney. The subgroup of 316 consecutive patients with clinically localized disease assessed in the current study are those patients of the 960 for which the pathological stage ranged from pT2A to pT3A; the minimum follow-up for censored patients was five years; RP was the primary treatment; and tissue blocks could be accessed from the RP specimens for use in gene expression profiling experiments. The date of PSA recurrence was defined as the date of the first increase in serum PSA ≥ 0.2 ng/mL after RP. These patients were randomly split into training and test sets. The test set was used solely for validation purposes. Differences in the distribution of the clinical variables between the training and test sets were evaluated by either a t-test, log-rank, or Chi-square test depending on whether the variable was continuous, time-to-event, or categorical. All statistical tests were two-sided and significance was defined as p<0.05.

### Gene expression profiling

Six µm sections from each of the FFPE tissue blocks were submitted to pathology review (JGK) and macrodissected to ensure > 30% malignant epithelium was used for total RNA extraction using the High Pure RNA Paraffin kit (Roche Diagnostics, Indianapolis, IN).

Gene expression profiling experiments were performed on all total RNA specimens in the training series with a 1200 gene custom designed DNA-mediated annealing, selection, ligation and extension microarray (DASL) (Illumina, San Diego, CA)(22). Both the gene expression profiles and the design of the DASL array can be accessed in the GEO database (accession pending). Three control genes, ALAS1, TUBA and ACTG1, were selected for the microarray and RTPCR analysis based on earlier studies in prostate cancer (12, 23).

Significance Analysis of Microarrays (SAM) analysis using the survival mode was used to measure prognostic significance for each probe on the array (24-26). The probes were ranked by the absolute value of the test statistic. False discovery rates were calculated by data permutation.

RTPCR assays were designed for the top-ranked prognostic marker candidates (n=30), including all genes under the lowest reported false discovery rates for both increasing and decreasing probes. Genes with a poor Pearson correlation (<0.4) between the array and RTPCR data among the training samples were excluded from further analysis.

### Construction of the prognostic model

Using the Memorial Sloan Kettering Cancer Centre on-line calculator (http://www.mskcc.org/mskcc/html/10088.cfm), the five-year nomogram recurrence score was calculated for each individual patient using the post-operative historical model. To select variables for a multivariate model, the delta CT values of the candidate genes from RTPCR as well as the predicted probabilities from the nomogram were processed by the L1 regularization path algorithm using the training samples (25). By cross-validating the training series using the path algorithm to set different limits on the potential for over-fitting the Cox model, the signature with the least error was selected. The final predictive model for deployment was built by fitting these selected variables to the training set using a Cox proportional-hazards model.

A cut-off for high and low risk stratification of the model, and a cut-off for the nomogram were both chosen under the assumption that the costs for false positives and false negatives are equivalent. Under this assumption, a cut-off giving the highest training set accuracy (defined by the total number of correctly classified patients) was chosen.

### Validation of the prognostic model

To evaluate the accuracy of predictive prognostic models with respect to the actual freedom from recurrence in the test set, a calibration curve was generated from the predicted 5-year recurrence-free probability estimated by Cox proportional-hazards regression and the Kaplan-Meier estimates of the actual recurrence-free probability at 5-years (24, 27). The performance of the final prognostic model in the test set was assessed by Kaplan-Meier curves and hazard ratios by stratifying the test set patients into a low risk and high risk group based on the pre-selected cut-off from the training set. All statistical analyses were performed in R, version 2.5.0 (www.r-project.org).

### RESULTS

### Patient characteristics

Total RNA was isolated from 316 prostatectomy FFPE tissues and 20 samples were excluded due to RNA degradation. The clinical and pathological characteristics of patients included in the training and test sets are summarized in Table 1. Median follow-up was 72 months, and median time from radical prostatectomy to biochemical recurrence was 34 months among those who recurred. Ninety-eight of 296 patients developed recurrence, including 74 patients who developed recurrence within 5 years of surgery. The training series consisted of 138 patients with the remaining 158 set aside for the test set. There was no statistically significant difference in the clinicopathological characteristics between these two sets of patients (Table 1).

### Gene expression and univariate analysis

The RNA samples from the training set of 138 patients were analysed by DASL array. The permutation of the SAM algorithm revealed false discovery rates of 0% for 20 genes on the DASL array. The top 30 genes as ranked by the score value from the SAM analysis had a false discovery rate of 6.8%. These 30 genes were then assessed by RTPCR analysis using the same training set. Six of the 30 selected genes displayed a correlation between DASL and RTPCR of less than 0.4 and thus were removed from further analysis. The effect of each gene on recurrence free probability was measured by Cox regression. The hazard ratio quantified the relative risk of PSA recurrence for each increase of 1 normalized CT. The hazard ratio and P value are recorded for both the training and test sets (Table 2). Twenty-three of 24 markers (except for marker HIST1H3D) continued to have a significant association to recurrence in the test set. In the same analysis, a 5-year postoperative nomogram was also a significant predictor of PSA recurrence in both the training and test sets (P value of .001 and .005, respectively).

### Example 2:

Further variable selection was performed on the RTPCR training set of Example 1 to build a multivariate prognostic classifier. Four variables were selected by the L1 Regularization algorithm: 3 genes (DPT, SSBP1 and MYH11) and the 5-year nomogram. These 4 variables were then modeled on the training set using Cox regression analysis.

### Classifier validation and survival analysis

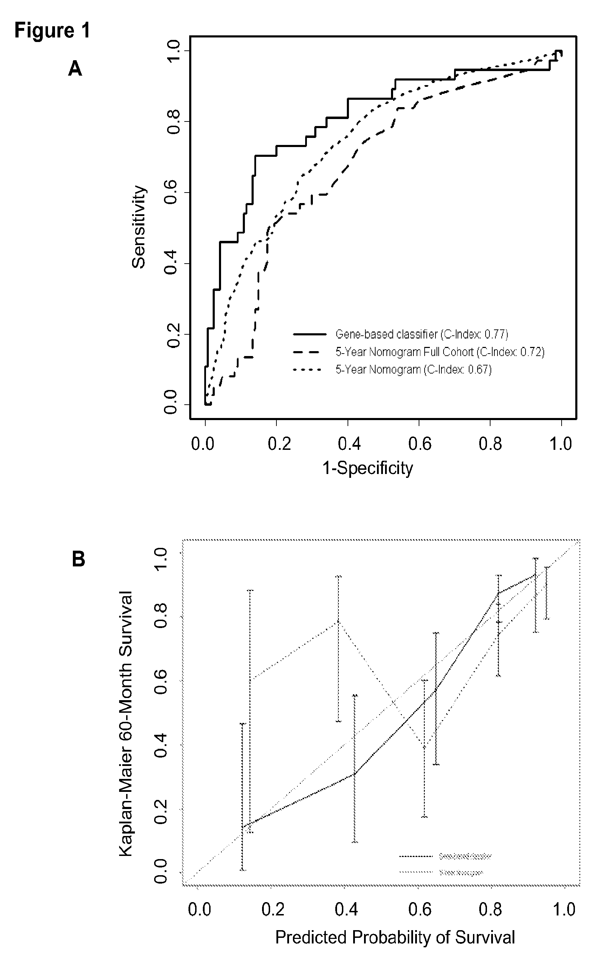

When testing the prognostic model on an independent test series of 157 patients, the c-index for the classifier was apparently higher than the c-index for the nomogram (0.77 vs. 0.67) (Figure 1A). The nomogram performance was consistent with published studies (c-index of 0.72) when tested on a consecutive prostate cancer patient cohort consisting of 960 patients from the same institution that was not limited to organ-confined disease (Figure 1A). We then used calibration curves to measure how close the 5-year predictive estimates on the test set were to the actual probabilities of recurrence. The classifier demonstrated a good calibration across the spectrum of predictions for the test set as compared to an ideal predictor, while the 5-year nomogram displayed less accuracy in detecting the more aggressive cases (Figure 1B).

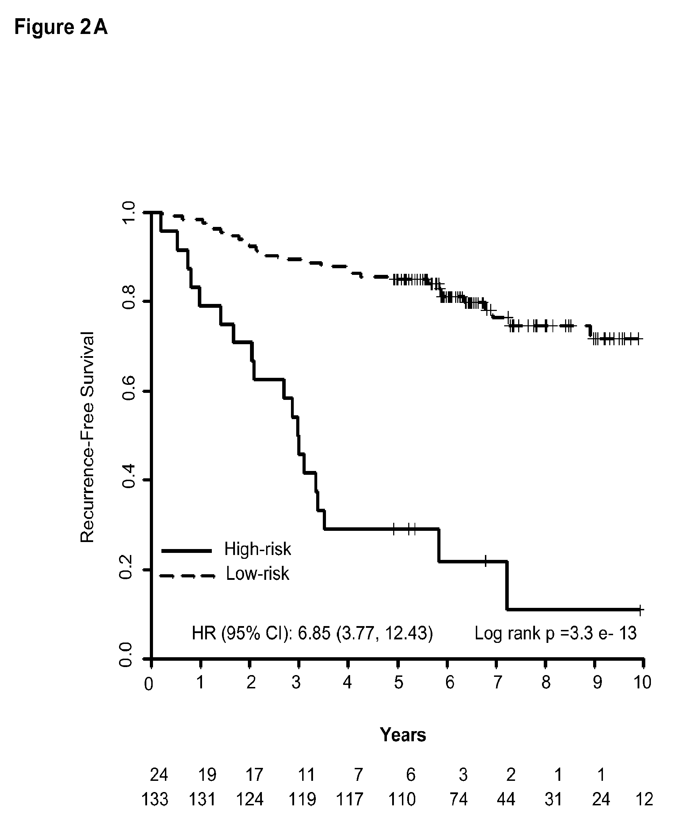

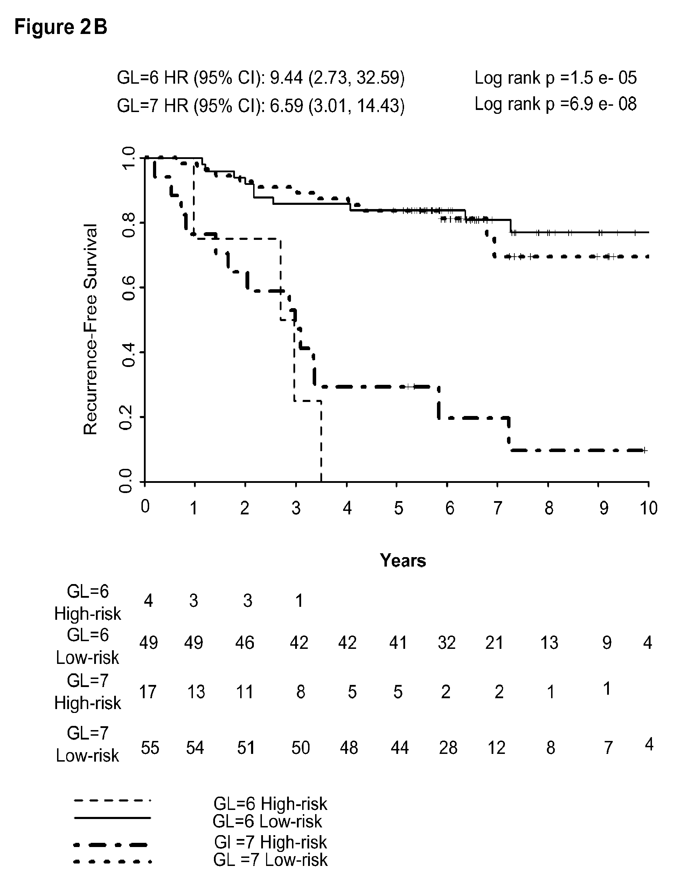

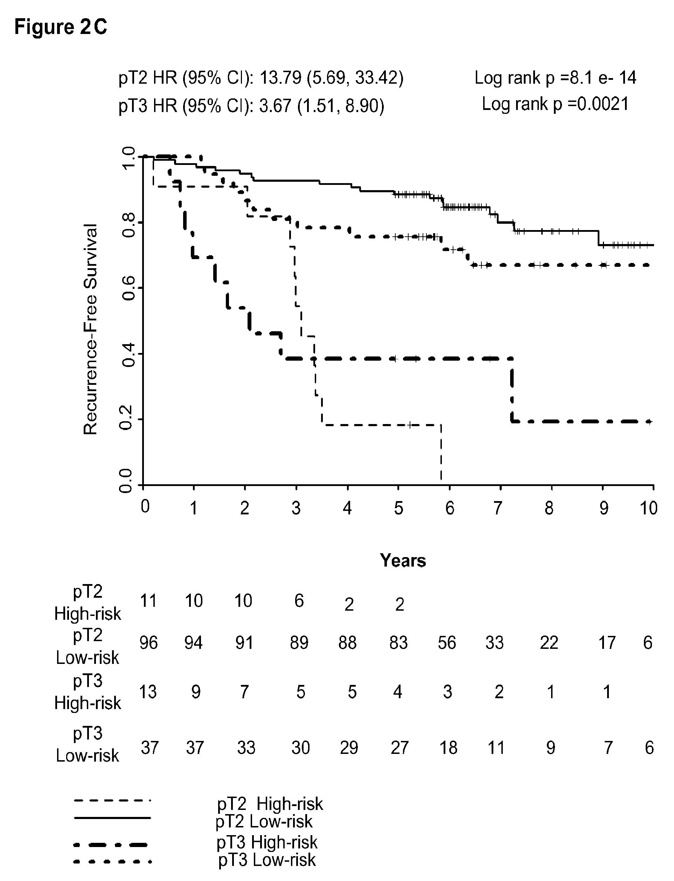

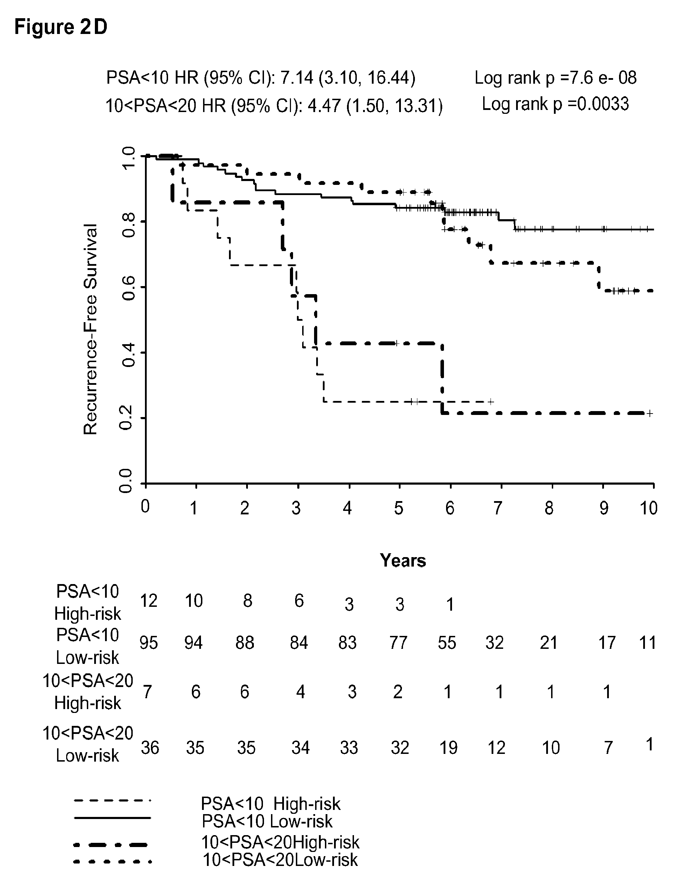

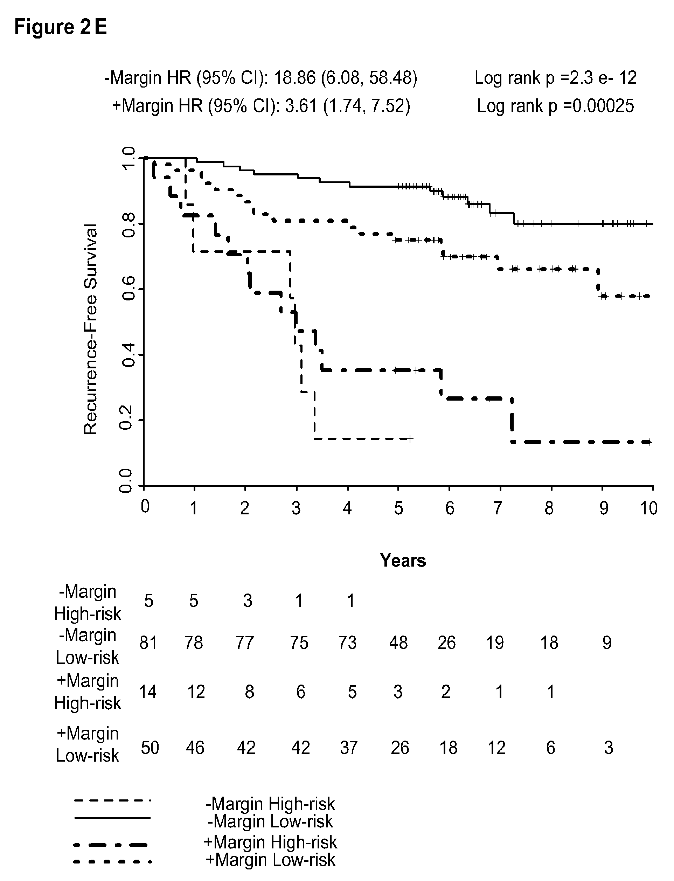

The cut-off from the training set was used to place test set patients into either a high- or low- risk group. Kaplan-Meier curves were generated for the test set samples by stratifying the patients into a low risk and high risk group based on a cutoff from the training set that produced the highest accuracy on the training samples. A calibration curve was generated from the predicted 5-year disease-free survival of the test set and the Kaplan-Meier estimates at 5-years based on cuts in the predicted probability at 0.3,0.5,0.7 and 0.9. The Kaplan-Meier analysis for PSA recurrence-free probability showed a highly significant difference in time to PSA recurrence for the predicted low- and high- risk groups (HR 6.85, 95% CI = 3.77 to 12.43, P < .001, Figure 2A). At 5 years, the absolute difference in PSA recurrence between the two groups was 58% (75% vs. 17 %). In addition, the classifier also represented a strong prognostic factor for PSA recurrence in the following subgroups of patients: Gleason score 6 or 7 (Figure 2B), pathological stage pT2 or pT3a (Figure 2C), pre-operative PSA concentration ≤10 ng/mL or 10<PSA≤20 ng/mL (Figure 2D), and positive or negative surgical margins (Figure 2E).

The clinical and pathological characteristics of patients included in the training and test sets are summarized in Table 1. Median follow-up was 72 months. Mean and median times from radical prostatectomy to biochemical recurrence were 40 and 34 months, respectively. Ninety-eight of 296 (33%) patients developed recurrence, including 74 (25%) patients who developed recurrence within 5 years of surgery.

### Application of an improved prognostic model

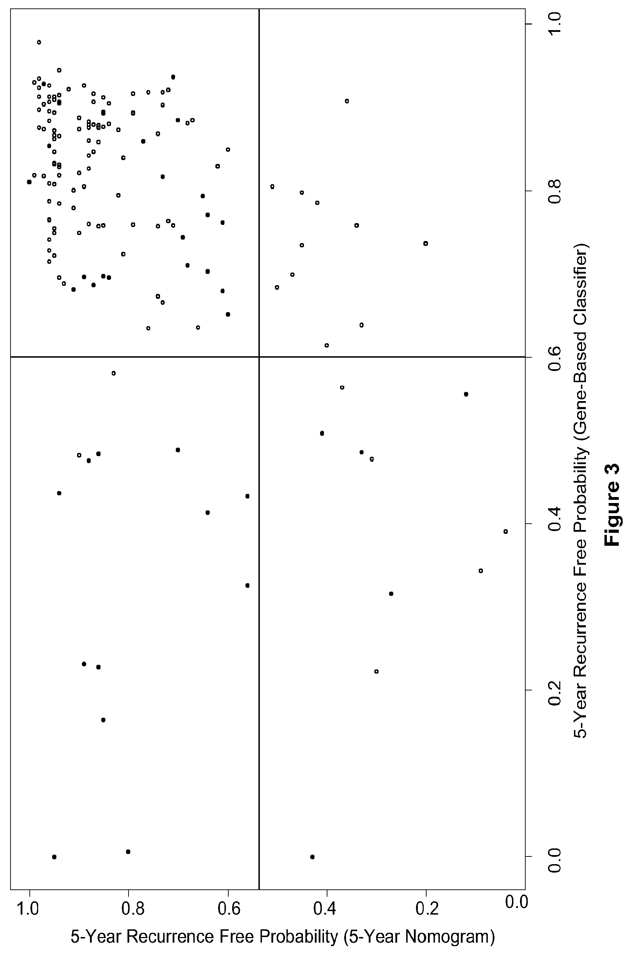

In order to evaluate the potential impact of the model on patient management, we compared the accuracy of prognostic stratification using the classifier compared to the 5-year post-operative nomogram on the test cohort. We used the cut-off based on each model's highest accuracy and then applied these to the test set (Figure 3). Within the 157 test set patients, 136 predicted as low risk by the nomogram had a recurrence rate of 23.5% (32/136). In comparison, when applied to this group, the classifier identified 14 patients as having a "high risk" of recurrence including 12 patients that had a documented recurrence (86%). Of the 122 patients for whom the classifier conferred "low risk" status, 20 patients (16.4%) experienced a PSA recurrence. Conversely, none of the 11 patients that were predicted as the "high risk" by the nomogram but "low risk" by the classifier had a documented recurrence. Thus the classifier conferred additional prognostic information to that provided by the postoperative nomogram on this series of prostate cancer patients.

### Example 3:

RTPCR assays were designed for the candidate markers and the three (3) control genes. Multiple PCR primers and probes were designed for the markers. CT values were normalized to the average of 3 control genes.

Each normalized gene was plotted against the DASL signal values. Total RNA was reverse 15 transcribed, and pre-amplified with the gene-specific primers. Pre-amplified cDNA was then quantified using ABI PRISM® 7900 sequence detection system (APPLIED BIOSYSTEMS).
DPT-1232 3096:
   Forward primer GGGTTGGAAGGATTTCCTGAA (SEQ ID NO 1)
   Reverse primer CCCTGCACTCATTTTCCTTACTG (SEQ ID NO2)
   Probe 5'Fam-3'MGB labeled probe TAGAAGACAAACGTTAGCATAC (SEQ ID NO 3)
MYH11-5893 460:
   Forward primer GCACTCAAGAGCAAGCTCAGAG (SEQ ID NO 4)
   Reverse primer TCGTTTCCTCGCCTGGTG (SEQ ID NO 5)
   Probe 5'Fam-3'MGB labeled probe AGGAAACTTCGCAGTGAT (SEQ ID NO 6)
SSBP1-291 2990:
   Forward primer AGTTTACCAACTGGGTGATGTCAG (SEQ ID NO 7)
   Reverse primer 5'Fam-3'MGB TTGATATGCCACGTCTCTGAGG (SEQ ID NO 8)
   labeled probe ATGGCACAGAATATCAG (SEQ ID NO 9)

### Example 4:

Also identified were additional panels, which while suboptimal compared to the preferred panel, are also useful in their own right. Each of the alternative panels can be used as a stand alone panel or in combination with the Kattan nomogram, the performance of which is improved by each of the alternative panels. The arrow symbol indicates the c-index corresponding to the model.
**Alt1:**
   FLNC+RASL12+MYLK+FLNA+NOM H5 -> 0.71
   FLNC+RASL12+MYLK+FLNA-> 0.67
**Alt2:**
   GAS1+SMTN+LMOD1+HIST1H3D+NOM_HS-> 0.74
   GAS1+SMTN+LMOD1+HIST1H3D-> 0.71
**Alt3:**
   SORBS1+PDLIM7+LIMS2+MT1X+NOM_H5 -> 0.73
   SORBS1+PDLIM7+LIMS2+MT1X-> 0.69

### Example 5:

Also identified were probes and primer pairs for the remaining markers. They are presented in Table 3.

### Example 6:

An alternative set of primers and probes were also developed for MYH11, DPT and SSBP1 as well as ALAS, ACTG and TUBA. The results were at least consistent or superior compared to the primer and probe sets of Example 3. They alternative primer and probes are:
DPT558F TGCAGTGGAAAGGGATCGC (SEQ ID NO 10)
DPT640R CCCAGATTTGGTATGTGGCA (SEQ ID NO 11)
DPT588P_FAM CATAATGTGCCGGATGACTGAATA (SEQ ID NO 12)
MYH 5895 F GCACTCAAGAGCAAGCTCAGA (SEQ ID NO 13)
MYH 5964R TAGAAGGAACGAAAGAGGTCTC (SEQ ID NO 14)
MYH 5925 P_Orange CCACAGGAAACTTCGCAGTGAT (SEQ ID NO 15)
SSBP1;281F GGGATAGTGAAGTTTACCAAC (SEQ ID NO 16)
SSBP1;359R TTGATATGCCACGTCTCTGA (SEQ ID NO 17)
SSBP1;312P (ORANGE) CAGTCAAAAGACAACATGGCACAG (SEQ ID NO 18)
TUBA 586 F TTCGCAAGCTGGCTGA (SEQ ID NO 19)
TUBA 666 R CATGAGCAGGGAGGTGAA (SEQ ID NO 20)
TUBA 639 P_Cy5 (Quasar) AGCTTTGGTGGGGGAACTGGTTCT (SEQ ID NO 21)
ALAS918F CAAGTGTCAGTCTGGTGCA (SEQ ID NO 22)
ALAS984R GTTGTTTCAAAGTGTCCATAAC (SEQ ID NO 23)
ALAS948P_FAM CTAGGAATGAGTCGCCACCCAC (SEQ ID NO 24)
ACTG 862 F AGCCTTCCTTCCTGGGTAT (SEQ ID NO 25)
ACTG 903 R TGATGGAGTTGAAGGTGGT (SEQ ID NO 26)
ACTG 882 P_Cy5 (Quasar) GAATCTTGCGGCATCCACGA (SEQ ID NO 27)

### DISCUSSION:

The relatively low level of complexity of the disclosed classifiers is also important with the ability to measure expression of a small set of genes in FFPE tissue with a diagnostic-approved platform. These significant advances address some key factors affecting the likelihood of successfully implementing this predictive tool in a clinical diagnostic setting. The need for very small concentrations of RNA derived from FFPE tissue will also facilitate its potential long-term applicability to routine pathology specimens including preoperative transrectal biopsies.

This systematic assessment of prostate cancer-related gene expression correlates of PSA recurrence in order to develop a gene-based classifier of recurrence in clinically localized prostate cancer of potential broad clinical utility. The novel gene predictors were identified using a custom DASL array, a microarray platform that allows high-throughput gene expression profiling of RNA derived from FFPE tissues (22). A key component of this study was the design of the custom DASL microarray gene set that is based on gene markers that were identified by re-analysis of published datasets (12, 13), in-house gene expression data (unpublished), and markers previously implicated in prostate cancer progression (14,16,17). This affords a degree of independent validation for those 30 genes that were most significant in this study. The 24-gene markers that correlated with PSA recurrence in the gene expression array analysis were further validated by RTPCR to produce a preferred 3-gene signature (DPT, MYH11 and SSBP1) that when combined with an established nomogram, resulted in a new classifier of PSA recurrence. This classifier was subsequently validated in an independent group of patients. DPT and MYH11 are novel prostate cancer prognostic markers while SSBP1 has previously been associated with aggressive prostate cancer (17).

An assessment of the value of this new classifier over a widely used nomogram for prostate cancer recurrence showed that the new classifier identified patients with both low- and high- risk of recurrence with much greater accuracy than the postoperative nomogram alone (9). The classifier presented here was also able to stratify patients within clinically relevant subgroups based on conventional clinicopathological parameters into high- and low risk- recurrence groups. Of note, the ability to stratify patients with Gleason 6 and 7 cancers represents a significant advance in predictive accuracy over current approaches. The use of PSA recurrence as a significant endpoint for prostate cancer has been disputed since only a proportion of patients who experience recurrence progress to clinically significant disease. These relationships will be more clearly defined as this cohort matures with data on metastases and death from PrCa. However, it is clear that the detection of a rising PSA post-prostatectomy is an important decision point when most physicians and patients consider further treatment options (5). In this context, the use of the 5-year nomogram to evaluate the potential impact of this classifier is valid with the majority of biochemical recurrences post-prostatectomy occurring within 5 years.

Of significance is the impact of this new classifier as a decision tool when considered against other published signatures and gene markers of molecular phenotype and prognosis in prostate cancer (12-20). This study is unique in being developed specifically to aid prediction of risk of recurrence in prostate cancer patients with clinically localized disease, since these patients represent >80% of newly diagnosed cases of prostate cancer in the United States. While published signatures and gene markers have been identified from cohorts of patients representing the spectrum of pathological stages, both the training and test cohorts in this study were restricted to organ-confined prostate cancer. The importance of having concordance between the patient group used in the development of a predictive tool, with the anticipated target group is reinforced by the relatively low performance of the 5-year nomogram in this cohort of clinically localized patients compared with previous reports. On further analysis, this is likely due to the limitation of assessing only organ-confined cases in this study. When tested on our consecutive prostate cancer patient cohort from the same institution that was not limited to organ-confined disease, the nomogram performance was consistent with previous studies (9, 11).

The relatively low level of complexity of the classifier is also important. With the ability to measure expression of a small set of genes in FFPE tissue and the use of a platform that is approved for diagnostic testing in archival specimens, it is a significant advance as it addresses some key factors affecting the likelihood of successfully implementing this predictive tool to a clinical diagnostic setting. The requirement for low concentrations of RNA derived from FFPE tissue will also facilitate its potential long-term applicability to routine pathology specimens including preoperative transrectal biopsies. Further validation in external cohorts of both surgical and preoperative biopsies, including replicating the gene selection in biopsies, is now required to confirm the wider applicability of this classifier in the preoperative setting.

The implementation of an accurate predictive classifier for localized prostate cancer has important implications for patient management of early prostate cancer. Patients with localized disease and high-risk features are likely to benefit from adjuvant therapies including hormone, radiation and systemic treatments and the benefits should be evaluated in treatment trials (7, 28, 29). Early phase clinical trials employing such agents are underway in the hormone-refractory setting but may ultimately be tested in localized prostate cancer as adjuvant therapies (30). Intrinsic to these studies is the accurate identification of high risk patients to ensure homogeneous patient groups8. Conversely, the improved identification of patients of low risk of recurrence will reduce the number of patients who are exposed to the morbidity of therapy as a result of the identification of increasing numbers of indolent cancers through PSA screening.

Finally, the development of an improved prognostic model for localized prostate cancer has the potential to facilitate better treatment decisions, either to forego treatment of indolent disease or offer adjuvant chemotherapy for men with high risk of recurrence.

### REFERENCES:

1. Ries LAG, Melbert D, Krapcho M et al: SEER Cancer Statistics Review, 1975-2005. Bethesda, MD: National Cancer Institute, posted to the SEER web site, 2008
2. Jemal A, Siegel R, Ward E et al: Cancer statistics, 2008. CA Cancer J Clin 2008; 58: 71.
3. Cooperberg MR, Moul JW and Carrol PR: The changing face of prostate cancer. J Clin Oncol 2005; 23: 8146.
4. Wilt TJ: SPCG-4: a needed START to PIVOTal data to promote and ProtecT evidence-based prostate cancer care. J Natl Cancer Inst 2008; 100: 1123.
5. Scardino PT: Localized prostate cancer is rarely a fatal disease. Nat Clin Pract Urol 2008; 5: 1.
6. Kumar S, Shelley M, Harrison C et al: Neo-adjuvant and adjuvant hormone therapy for localised and locally advanced prostate cancer. Cochrane Database Syst Rev 2006: CD006019.
7. Van der Kwast TH, Bolla M, Van Poppel H et al: Identification of patients with prostate cancer who benefit from immediate postoperative radiotherapy: EORTC 22911. J Clin Oncol 2007; 25: 4178.
8. Shariat SF, Karakiewicz PI, Suardi N et al: Comparison of nomograms with other methods for predicting outcomes in prostate cancer: a critical analysis of the literature. Clin Cancer Res 2008; 14: 4400.
9. Graefen M, Karakiewicz PI, Cagiannos I et al: Validation study of the accuracy of a postoperative nomogram for recurrence after radical prostatectomy for localized prostate cancer. J Clin Oncol 2002; 20: 951.
10. Stephenson AJ, Scardino PT, Eastham JA et al: Postoperative nomogram predicting the 10-year probability of prostate cancer recurrence after radical prostatectomy. J Clin Oncol 2005; 23: 7005.
11. Kattan MW, Wheeler TM and Scardino PT: Postoperative nomogram for disease recurrence after radical prostatectomy for prostate cancer. J Clin Oncol 1999; 17: 1499.
12. Glinsky GV, Glinskii AB, Stephenson AJ et al: Gene expression profiling predicts clinical outcome of prostate cancer. J Clin Invest 2004; 113: 913.
13. Henshall SM, Afar DE, Hiller J et al: Survival analysis of genome-wide gene expression profiles of prostate cancers identifies new prognostic targets of disease relapse. Cancer Res 2003; 63: 4196.
14. Lapointe J, Li C, Higgins JP et al: Gene expression profiling identifies clinically relevant subtypes of prostate cancer. Proc Natl Acad Sci USA 2004; 101: 811.
15. Nakagawa T, Kollmeyer TM, Morlan BW et al: A tissue biomarker panel predicting systemic progression after PSA recurrence post-definitive prostate cancer therapy. PloS ONE 2008; 3: e2318.
16. Singh D, Febbo PG, Ross K et al: Gene expression correlates of clinical prostate cancer behavior. Cancer Cell 2002; 1: 203.
17. Yu YP, Landsittel D, Jing L et al: Gene expression alterations in prostate cancer predicting tumor aggression and preceding development of malignancy. J Clin Oncol 2004; 22: 2790.
18. Setlur S, Mertz K, Hoshida Y et al: Estrogen-dependent signaling in a molecularly distinct subclass of aggressive prostate cancer. J Natl Cancer Inst 2008; 100: 815.
19. Henshall S, Horvath L, Quinn D et al: Zinc-alpha2-glycoprotein expression as a predictor of metastatic prostate cancer following radical prostatectomy. J Natl Cancer Inst 2006; 98: 1420.
20. Mucci L, Pawitan Y, Demichelis F et al: Testing a multigene signature of prostate cancer death in the Swedish Watchful Waiting Cohort. Cancer Epidemiol Biomarker Prev 2008; 17: 1682.
21. Quinn DI, Henshall SM and Sutherland RL: Molecular markers of prostate cancer outcome. Eur J Cancer 2005; 41: 858.
22. Bibikova M, Talantov D, Chudin E et al: Quantitative gene expression profiling in formalin-fixed,paraffin-embedded tissues using universal bead arrays. Am J Pathol 2004; 165: 1799.
23. Ohl F, Jung M, Xu C et al: Gene expression studies in prostate cancer tissue: which reference gene should be selected for normalization? J Mol Med 2005; 83: 1014.
24. Harrell Jr FE: Regression modeling strategies: with applications to linear models, logistic regression, and survival analysis. New York: Springer-Verlag New York, 2001
25. Park MY and Hastie T: L1 regularization path algorithm for generalized linear models. J R Stat Soc: Series B (Statistical Methodology) 2007; 69: 659.
26. Tusher VG, Tibshirani R and Chu G: Significance analysis of microarrays applied to the ionizing radiation response. Proc Natl Acad Sci USA 2001; 98: 5116.
27. Team RDC: R: a language and environment for statistical computing. Vienna: R Foundation for Statistical Computing, 2007
28. Joniau S and Van Poppel H: Localized prostate cancer: can we better define who is at risk of unfavourable outcome? BJU International 2008; 101: S5.
29. Bolla M, van Poppel H, Collette L et al: Postoperative radiotherapy after radical prostatectomy: a randomised controlled trial (EORTC trial 22911). Lancet 2005; 366: 572.
30. Mazhar D and Waxman J: Early chemotherapy in prostate cancer. Nat Clin Pract Urol 2008; 5: 486.

**Table 1. Patient Characteristics of the Test and Training Cohorts.**

| | ***Training Cohort*** | ***Test Cohort*** | |
|---|---|---|---|
| ***Characteristic*** | **(*no.* of *patients* = *138)*** | **(*no.* of *patients* = *158)*** | ***P Value*** |
| Age, years | | | |
| <60 | 58 | 56 | .50† |
| ≥60 | 80 | 102 | |
| Gleason Score | | | |
| ≤6 | 57 | 75 | .17* |
| 7 | 64 | 72 | |
| 8-10 | 17 | 10 | |
| Unknown | 0 | 1 | |
| Clinical Stage | | | |
| T1 | 56 | 67 | .75* |
| T2 | 80 | 90 | |
| T3 | 2 | 1 | |
| pT Stage | | | |
| pT2a | 4 | 13 | .07* |
| pT2b | 13 | 14 | |
| pT2c | 61 | 81 | |
| pT3a | 60 | 50 | |
| PSA at diagnosis | | | |
| <10 | 97 | 107 | .55† |
| 10<PSA≤20 | 34 | 43 | |
| >20 | 7 | 8 | |
| Extracapsular | | | |
| Extension | | | |
| Capsular Invasion | 64 | 84 | .13* |
| Focal | 51 | 39 | |
| Established | 9 | 12 | |
| None | 14 | 23 | |
| Margins | | | |
| Positive | 53 | 70 | .36* |
| Negative | 85 | 88 | |
| Adjuvant Treatment | | | |
| Yes | 12 | 13 | 1.00* |
| No | 126 | 145 | |
| Outcome | | | |
| Disease-Free | 89 | 109 | .40‡ |
| PSA recurrence | 49 | 48 | |
| Clinical (Local/Distant) | 0 | 1 | |
| Outcome at 5 Years | | | |
| Disease-Free | 101 | 121 | .47‡ |
| PSA recurrence | 37 | 36 | |
| Clinical (Local/Distant) | 0 | 1 | |

| | | | |
|---|---|---|---|
| * The P value was calculated by the chi-square test. † The P value was calculated by the t-test with the characteristic assessed as a continuous variable. ‡ The P value was calculated by the log-rank test. | | | |

**Table 2. Cox regression for each tested RTPCR marker in the Test and Training cohorts.**

| *MARKER* | *Training Hazard Ratio †* | *Training P Value** | *Test Hazard Ratio †* | *Test P Value* * | *DESCRIPTION* |
|---|---|---|---|---|---|
| ACTG2 | 1.6 | < .001 | 1.35 | < .001 | Actin, gamma 2 |
| CALD1 | 1.37 | .007 | 1.44 | .003 | Caldesmon 1 |
| CBX3 | 0.53 | .05 | 0.64 | .02 | Chromobox homolog 3 |
| DCHS1 | 1.52 | .004 | 1.66 | < .001 | Dachsous 1 |
| DKK3 | 1.53 | .002 | 1.75 | < .001 | Dickkopf homolog 3 |
| DPT | 1.48 | < .001 | 1.20 | < .001 | Dermatopontin |
| FLNA | 1.31 | .004 | 1.43 | .005 | Filamin A, alpha |
| FLNC | 1.65 | < .001 | 1.50 | < .001 | Filamin C, gamma |
| GAS1 | 1.43 | < .001 | 1.59 | < .001 | Growth arrest-specific 1 |
| GSN | 1.63 | .003 | 2.02 | .001 | Gelsolin |
| HIST1H3D | 0.75 | .008 | 0.89 | .20 | Histone 1, H3d |
| LIMS2 | 1.75 | < .001 | 1.76 | < .001 | LIM and senescent cell antigen-like domains 2 |
| LMOD1 | 1.80 | < .001 | 1.57 | < .001 | Leiomodin 1 |
| MT1X | 1.56 | .001 | 2.06 | < .001 | Metallothionein 1X |
| MYH11 | 1.68 | < .001 | 1.29 | < .001 | Myosin, heavy polypeptide 11 |
| MYLK | 1.73 | < .001 | 1.56 | < .001 | Myosin, light polypeptide kinase |
| PDLIM3 | 1.37 | .003 | 1.33 | .002 | PDZ and LIM domain 3 |
| PDLIM7 | 1.92 | < .001 | 1.47 | .01 | PDZ and LIM domain 7 |
| RASL12 | 1.84 | < .001 | 2.09 | < .001 | RAS-like, family 12 |
| SH3BGRL | 1.32 | .04 | 1.81 | < .001 | SH3 domain binding glutamic acid-rich protein like |
| SMTN | 1.89 | < .001 | 1.78 | < .001 | Smoothelin |
| SORBS1 | 1.68 | < .001 | 1.40 | .002 | Sorbin and SH3 domain containing 1 |
| SSBP1 | 0.34 | .02 | 0.32 | < .001 | Single-stranded DNA binding protein 1 |
| TNS1 | 1.88 | < .001 | 1.70 | .001 | Tensin 1 |

| | | | | | |
|---|---|---|---|---|---|
| * Cox regression p-value † The hazard ratio is for each increase of 1 in the normalized CT value. | | | | | |

**Table 3. Primer pairs and probes for the markers.**

| | **SEQ ID** | **MARKER NAME** | **OLIGONUCLEOTIDE** | **5'-3' SEQUENCE** |
|---|---|---|---|---|
| **1** | SEQ ID 28 | ACTG1 | 5'Fam-3'MGB labeled probe | TTGCGGCATCCAC |
| | SEQ ID 29 | ACTG1 | Forward primer | CAGCCTTCCTTCCTGGGTATG |
| | SEQ ID 30 | ACTG1 | Reverse primer | CATGATGGAGTTGAAGGTGGTCT |
| **2** | SEQ ID 31 | ACTG2 | 5'Fam-3'MGB labeled probe | CATGAGACAACCTACAATT |
| | SEQ ID 32 | ACTG2 | Forward primer | TTTATTGGCATGGAGTCCGC |
| | SEQ ID 33 | ACTG2 | Reverse primer | CCTTACGGATGTCAATGTCACACT |
| **3** | SEQ ID 34 | ALAS1 | 5'Fam-3'MGB labeled probe | CAGTATGATCGTTTCTTTGAG |
| | SEQ ID 35 | ALAS1 | Forward primer | ATAACTTGCCAAAATCTGTTTCCACT |
| | SEQ ID 36 | ALAS1 | Reverse primer | AAACTCGATAGGTGTGGTCATTCTT |
| **4** | SEQ ID 37 | CALD1 | 5'Fam-3'MGB labeled probe | ATGCCTGATGACCTATAA |
| | SEQ ID 38 | CALD1 | Forward primer | CATGGCAGATAGGTATCAATATGTT TTC |
| | SEQ ID 39 | CALD1 | Reverse primer | TCAACTCCTTCTAACAGTTCTAATCT CTCT |
| **5** | SEQ ID 40 | CBX3 | 5'Fam-3'MGB labeled probe | ATTTGCCAGAGGTCTTGAT |
| | SEQ ID 41 | CBX3 | Forward primer | AAAGAGATGCTGCTGACAAACCA |
| | SEQ ID 42 | CBX3 | Reverse primer | CATCAATTCTCCACTGCTGTCTG |
| **6** | SEQ ID 43 | DCHS1 | 5'Fam-3'MGB labeled probe | TGAACAGCTCAACAGGG |
| | SEQ ID 44 | DCHS1 | Forward primer | GCCGTGAGGCATTTGCA |
| | SEQ ID 45 | DCHS1 | Reverse primer | CACTCGCGCACGCAACT |
| **7** | SEQ ID 46 | DKK3 | 5'Fam-3'MGB labeled probe | CAGACTGGACAAATGG |
| | SEQ ID 47 | DKK3 | Forward primer | CGAGAAATTCACAAGATAACCAACA |
| | SEQ ID 48 | DKK3 | Reverse primer | CTGCCTTCTTCGTCTCCCAC |
| **8** | SEQ ID 3 | DPT | 5'Fam-3'MGB labeled probe | TAGAAGACAAACGTTAGCATAC |
| | SEQ ID 1 | DPT | Forward primer | GGGTTGGAAGGATTTCCTGAA |
| | SEQ ID 2 | DPT | Reverse primer | CCCTGCACTCATTTTCCTTACTG |
| **9** | SEQ ID 49 | FLNA | 5'Fam-3'MGB labeled probe | ATGGCCCAAGGAC |
| | SEQ ID 50 | FLNA | Forward primer | CAGCAAAGCAGGCAACAACAT |
| | SEQ ID 51 | FLNA | Reverse primer | CGTGCTTCACCAGGATCTCC |
| **10** | SEQ ID 52 | FLNC | 5'Fam-3'MGB labeled probe | CAACCCCAGAGTTTTAAGGA |
| | SEQ ID 53 | FLNC | Forward primer | GGTCTGGTCTCTCTGGTGGCT |
| | SEQ ID 54 | FLNC | Reverse primer | TTCTCTGATTGTGCTTTCCTTTCC |
| **11** | SEQ ID 55 | GAS1 | 5'Fam-3'MGB labeled probe | TATAGAATCCATTTGTCATCAGG |
| | SEQ ID 56 | GAS1 | Forward primer | ACTCACATCCATATTACACCTTTCCC |
| | SEQ ID 57 | GAS1 | Reverse primer | TAAATATAGCACACTTCACAATGGACTG T |
| **12** | SEQ ID 58 | GSN | 5'Fam-3'MGB labeled probe | CCGAGTTCCTCAAGGC |
| | SEQ ID 59 | GSN | Forward primer | GCGGCCCAACAGCATG |
| | SEQ ID 60 | GSN | Reverse primer | TGCAGGCCAGGCTCCTT |
| **13** | SEQ ID 61 | HIST1H3D | 5'Fam-3'MGB labeled probe | AAGTTCGCAATGGCTCGTA |
| | SEQ ID 62 | HIST1H3D | Forward primer | CAAGGCCAAGGCAGGTTTTAG |
| | SEQ ID 63 | HIST1H3D | Reverse primer | CACCCGTGGACTTGCGAG |
| **14** | SEQ ID 64 | LIMS2 | 5'Fam-3'MGB labeled probe | TCCACACCCACAAGC |
| | SEQ ID 65 | LIMS2 | Forward primer | CACACTGAGCCAGCAAGTCCT |
| | SEQ ID 66 | LIMS2 | Reverse primer | TTCCGAAGGATGGAGGTGG |
| **15** | SEQ ID 67 | LMOD1 | 5'Fam-3'MGB labeled probe | CTGAACTGTGAGTCCTGAT |
| | SEQ ID 68 | LMOD1 | Forward primer | GCTGTGCCCCACCTGTTG |
| | SEQ ID 69 | LMOD1 | Reverse primer | TAGAGTCCTCCAGGGAGCCC |
| **16** | SEQ ID 70 | MT1X | 5'Fam-3'MGB labeled probe | CTCGAAATGGACCCCAAC |
| | SEQ ID 71 | MT1X | Forward primer | GATCGGGAACTCCTGCTTCTC |
| | SEQ ID 72 | MT1X | Reverse primer | CAGGAGCCAACAGGCGAG |
| **17** | SEQ ID 6 | MYH11 | 5'Fam-3'MGB labeled probe | AGGAAACTTCGCAGTGAT |
| | SEQ ID 4 | MYH11 | Forward primer | GCACTCAAGAGCAAGCTCAGAG |
| | SEQ ID 5 | MYH11 | Reverse primer | TCGTTTCCTCGCCTGGTG |
| **18** | SEQ ID 73 | MYLK | 5'Fam-3'MGB labeled probe | TCTGAAGAAGATGTGTCCCA |
| | SEQ ID 74 | MYLK | Forward primer | CCAGCCCGCTCAATGC |
| | SEQ ID 75 | MYLK | Reverse primer | CTCAGCAACAGCCTCAAGGAA |
| **19** | SEQ ID 76 | PDLIM3 | 5'Fam-3'MGB labeled probe | GAAGATCACACCTTTTAATG |
| | SEQ ID 77 | PDLIM3 | Forward primer | GGATAATGGCAAGCCACTCATAA |
| | SEQ ID 78 | PDLIM3 | Reverse primer | TCTGTTTCTCTCCTTCTCTCTTCCA |
| **20** | SEQ ID 79 | PDLIM7 | 5'Fam-3'MGB labeled probe | CTGAAGATGACCTGGCACG |
| | SEQ ID 80 | PDLIM7 | Forward primer | GAAGAAGATTACAGGCGAGATCATG |
| | SEQ ID 81 | PDLIM7 | Reverse primer | CAGGCAGCACAGGTAAAGCA |
| **21** | SEQ ID 82 | RASL12 | 5'Fam-3'MGB labeled probe | CTTCCCGACCCACAGGCCAGCT |
| | SEQ ID 83 | RASL12 | Forward primer | ACCACATGCTTGCAGTCCTACA |
| | SEQ ID 84 | RASL12 | Reverse primer | AGTGGCCTGGAGCAAAAGTG |
| **22** | SEQ ID 85 | SH3BGRL | 5'Fam-3'MGB labeled probe | CTAGCAAAGAGATTAGACTTT |
| | SEQ ID 86 | SH3BGRL | Forward primer | CATGAAGTGGGATGCCAAGTAA |
| | SEQ ID 87 | SH3BGRL | Reverse primer | GATCGCCAACCTGTTTTATAAGAGT |
| **23** | SEQ ID 88 | SMTN | 5'Fam-3'MGB labeled probe | TTCACCTATGTGCAGTCG |
| | SEQ ID 89 | SMTN | Forward primer | GCAAGAAGCCTGACCCCAA |
| | SEQ ID 90 | SMTN | Reverse primer | TCGTGGCGTCGCAGGT |
| **24** | SEQ ID 91 | SORBS1 | 5'Fam-3'MGB labeled probe | CTTTAATGGTGATACACAAGTAGA |
| | SEQ ID 92 | SORBS1 | Forward primer | CCAGTGCAGGTTTTGGAATATG |
| | SEQ ID 93 | SORBS1 | Reverse primer | TGATCCTCTCACCCTTTCTGAAG |
| **25** | SEQ ID 9 | SSBP1 | 5'Fam-3'MGB labeled probe | ATGGCACAGAATATCAG |
| | SEQ ID 7 | SSBP1 | Forward primer | AGTTTACCAACTGGGTGATGTCAG |
| | SEQ ID 8 | SSBP1 | Reverse primer | TTGATATGCCACGTCTCTGAGG |
| **26** | SEQ ID 94 | TNS1 | 5'Fam-3'MGB labeled probe | CACGGCATCCCCAAC |
| | SEQ ID 95 | TNS1 | Forward primer | AAGCCCTTGTTTCTGCACCA |
| | SEQ ID 96 | TNS1 | Reverse primer | GCCGACATCCTCCTTTAGACTC |
| **27** | SEQ ID 97 | TUBA | 5'Fam-3'MGB labeled probe | CGGGCTGTGTTTGTAGA |
| | SEQ ID 98 | TUBA | Forward primer | GACTCCTTCAACACCTTCTTCAGTG |
| | SEQ ID 99 | TUBA | Reverse primer | TGCGAACTTCATCAATGACTGTG |

## Claims

1. A method for distinguishing between subjects in a high recurrence risk category of prostate cancer and subjects in a low recurrence risk category of prostate cancer, comprising:
(i) measuring an expression level of at least two diagnostic markers selected from the set of markers, except for Histone 1 H3d, presented in TABLE 2, with each expression level measured relative to an average of expression levels of two or more control markers and
(ii) combining the expression level of the at least two diagnostic markers with
a predictive probability based on a Kattan nomogram, to assign to subjects one of the high recurrence risk category of prostate cancer and the low recurrence risk category of prostate cancer.

2. The method of claim 1, wherein the Kattan nomogram is a composite measure based on two or more members of a group consisting of PSA value prior to surgery (and prior to hormone therapy, if received), PSA at the time of prostatectomy, primary Gleason at surgery, secondary Gleason at surgery, prostatectomy Gleason Sum, the year of prostatectomy, the months disease free, whether or not surgical margins were positive, whether or not cancer was found in seminal vesicles, whether or not there was extra-capsular extension, whether or not cancer was found in lymph nodes (if any were removed), pre-radiotherapy PSA, radiation dose (if applicable), whether surgical margins were positive or negative (if applicable), whether there was seminal vesicle involvement (if applicable), whether there was lymph node involvement (if applicable), whether there was extra capsular involvement (if applicable), whether or not neo-adjuvant hormones were prescribed, whether or not neo-adjuvant radiation was prescribed.

3. The method of claim 1, wherein the Kattan nomogram is a composite measure based on two or more members of a group consisting of pretreatment PSA level, combined Gleason grade, specimen Gleason sum, clinical stage, surgical margin status, prostatic capsular invasion maximum cancer length in a core, total length of cancer in the biopsy cores, percent of cores positive level, extraprostatic extension, level of extraprostatic extension, apoptotic index, percent of cancer in one or more cores, percent of high grade cancer in one or more cores, total tumor volume, zone of location of the cancer, presence of seminal vesicle invasion, type of seminal vesicle invasion, p53, Ki-67, p27, DNA ploidy status, lymph node status, and lymphovascular invasion.

4. The method of claim 1, wherein the classifier comprises diagnostic markers MYH11, SSBP1 and DPT.

5. The method of claim 4, wherein the classifier comprises diagnostic markers other than Histone 1 H3d, selected from TABLE 2, in addition to MYH11, SSBP1 and DPT.

6. The method of claim 1, wherein the control markers are TUBA, ALAS1 and ACTG1.

7. The method of claim 1, wherein control markers are selected for relatively steady expression levels as detected by RT-PCR.

8. A method of predicting the recurrence of prostate cancer comprising:
determining an expression level of DPT relative to a standard;
determining expression level of at least one additional markers from TABLE 2; and
transforming the expression level of DPT and the expression level of at least one additional markers, except for Histone 1 H3d, from TABLE 2 into a score corresponding to a probability of recurrence of prostate cancer.

9. The method of claim 8 further comprising as an alternative to the step of transforming the step of combining the expression level of DPT and the expression level of at least one additional markers, except for Histone 1 H3d, from TABLE 2 with at least one additional indicator of prostate cancer recurrence to determine a composite score.

10. The method of claim 9 wherein the composite score reflects a likelihood of recurrence of prostate cancer.

11. The method of claim 8, wherein the expression level of DPT and the expression level of at least one additional markers, except for Histone 1 H3d, from TABLE 2 is determined prior to at least one treatment selected from the group consisting of prostectomy, hormone therapy, single agent chemotherapy, two agent chemotherapy and treatment with a farnesyl transferase inhibitor.

12. A kit for detecting an expression level of at least DPT, the kit comprising reagents and a first probe set comprising a first probe specifically recognizing DPT and a second probe specifically recognizing a second Marker, except for Histone 1 H3d, selected from TABLE 2, wherein the kit comprises reagents necessary for RT PCR.

13. The use of a kit in the method of any one of claims 8-11, the kit comprising reagents and a first probe set comprising a first probe specifically recognizing DPT and a second probe specifically recognizing a second Marker, except for Histone 1 H3d, selected from TABLE 2.

14. The kit of claim 12 or the use of claim 13, further comprising a device for converting the expression level of DPT and the expression level of at least one additional markers, except for Histone 1 H3d, from TABLE 2 into an indicator of a likelihood of recurrence of prostate cancer.

15. The kit of claim 12 or the use of claim 13, wherein the device is a nomogram.

16. The kit or use of claim 15 wherein the nomogram is implemented as one or more members of the group consisting of a mechanical device, a graphical representation, and software instructions to implement a user interface for providing a representation of the indicator of the likelihood of recurrence of prostate cancer.

## Patentansprüche

1. Verfahren zur Unterscheidung zwischen Individuen in einer Kategorie mit hohem Rezidivrisiko für Prostatakrebs und Individuen in einer Kategorie mit geringem Rezidivrisiko für Prostatakrebs, umfassend:
(i) Messen eines Expressionsniveaus von mindestens zwei Diagnosemarkern, ausgewählt aus dem in TABELLE 2 dargestellten Satz von Markern mit Ausnahme von Histon 1 H3d, wobei jedes Expressionsniveau relativ zu einem Durchschnitt der Expressionsniveaus von zwei oder mehreren Kontrollmarkern gemessen wird; und
(ii) Kombinieren des Expressionsniveaus der mindestens zwei Diagnosemarker mit
einer Vorhersagewahrscheinlichkeit auf der Basis eines Kattan-Nomogramms, um die Individuen der Kategorie mit hohem Rezidivrisiko für Prostatakrebs oder der Kategorie mit geringem Rezidivrisiko für Prostatakrebs zuzuordnen.

2. Verfahren nach Anspruch 1, wobei das Kattan-Nomogramm ein zusammengesetztes Maß ist, das auf zwei oder mehr Mitgliedern einer Gruppe beruht, bestehend aus dem PSA-Wert vor der Operation (und vor der Hormontherapie, sofern empfangen), PSA während der Prostatektomie, dem primären Gleason-Score bei der Operation, dem sekundären Gleason-Score bei der Operation, der Prostatektomie-Gleason-Summe, dem Jahr der Prostatektomie, den krankheitsfreien Monaten, und zwar je nachdem ob die Resektionsränder positiv waren oder nicht, ob Krebs in Samenblasen festgestellt wurde oder nicht, ob extrakapsuläre Ausbreitung bestand oder nicht, ob Krebs in Lymphknoten gefunden wurde oder nicht (wenn überhaupt welche entfernt wurden), Prä-Strahlentherapie PSA, Strahlungsdosis (falls zutreffend), ob Resektionsränder positiv oder negativ waren (falls zutreffend), ob Samenblasenbeteiligung bestand (falls zutreffend), ob Lymphknotenbeteiligung bestand (falls zutreffend), ob zusätzliche Kapselbeteiligung bestand (falls zutreffend), ob neoadjuvante Hormone verschrieben wurden oder nicht, ob neo-adjuvante Strahlentherapie verschrieben wurde oder nicht.

3. Verfahren nach Anspruch 1, wobei das Kattan-Nomogramm ein zusammengesetztes Maß ist, das auf zwei oder mehreren Mitgliedern einer Gruppe beruht, bestehend aus dem Vorbehandlungs-PSA-Wert, kombiniertem Gleason-Score, Proben-Gleason-Summe, klinischem Stadium, Resektionsränder-Status, Invasion der Prostatakapsel, maximaler Krebslänge in einem Kern, Gesamtlänge von Krebs in den Biopsie-Kernen, Prozent der positiven Kerne, extraprostatischer Ausbreitung, Ausmaß der extraprostatischen Ausbreitung, apoptotischem Index, Prozent Krebs in einem oder mehreren Kernen, Prozent an hochgradigem Krebs in einem oder mehreren Kernen, Gesamttumorvolumen, Zone der Stelle der Krebserkrankung, Vorhandensein von Samenblasen-Invasion, Art der Samenblasen-Invasion, p53, Ki-67, p27, DNA-Ploidie-Status, Lymphknotenstatus und Lymphgefäßinvasion.

4. Verfahren nach Anspruch 1, wobei der Klassifikator die Diagnosemarker MYH11, SSBP1 und DPT umfasst.

5. Verfahren nach Anspruch 4, wobei der Klassifikator aus TABELLE 2 ausgewählte Diagnosemarker mit Ausnahme von Histon 1 H3d, zusätzlich zu MYH11, SSBP1 und DPT umfasst.

6. Verfahren nach Anspruch 1, wobei die Kontrollmarker TUBA, ALAS1 und ACTG1 sind.

7. Verfahren nach Anspruch 1, wobei die Kontrollmarker für relativ konstante Expressionsniveaus ausgewählt werden, wie durch RT-PCR nachgewiesen.

8. Verfahren zur Vorhersage des Rezidivs von Prostatakrebs, umfassend:
Bestimmen eines Expressionsniveaus von DPT relativ zu einem Standard;
Bestimmen des Expressionsniveaus von mindestens einem zusätzlichen Marker aus TABELLE 2; und
Umwandeln des Expressionsniveaus von DPT und des Expressionsniveaus von mindestens einem zusätzlichen Marker, mit Ausnahme von Histon 1 H3d aus TABELLE 2, in einen Score, der der Wahrscheinlichkeit des Rezidivs von Prostatakrebs entspricht.

9. Verfahren nach Anspruch 8, das ferner als Alternative zu dem Umwandlungsschritt den Schritt Kombinieren des Expressionsniveaus von DPT und des Expressionsniveaus von mindestens einem zusätzlichen Marker mit Ausnahme von Histon 1 H3d aus TABELLE 2, mit mindestens einem zusätzlichen Indikator für Prostatakrebsrezidiv zur Bestimmung eines zusammengesetzten Scores umfasst.

10. Verfahren nach Anspruch 9, wobei der zusammengesetzte Score eine Wahrscheinlichkeit eines Rezidivs von Prostatakrebs wiederspiegelt.

11. Verfahren nach Anspruch 8, wobei das Expressionsniveau von DPT und das Expressionsniveau von mindestens einem zusätzlichen Marker, mit Ausnahme von Histon 1 H3d, aus TABELLE 2, vor mindestens einer Behandlung, ausgewählt aus der Gruppe, bestehend aus Prostatektomie, Hormontherapie, Chemotherapie mit einem Arzneimittel, Chemotherapie mit zwei Arzneimitteln und Behandlung mit einem Farnesyltransferaseinhibitor, bestimmt wird.

12. Kit zum Nachweisen eines Expressionsniveaus von mindestens DPT, wobei das Kit Reagenzien und einen ersten Sondensatz umfasst, umfassend eine erste Sonde, die spezifisch DPT erkennt, und eine zweite Sonde, die spezifisch einen zweiten Marker erkennt, mit Ausnahme von Histon 1 H3d, ausgewählt aus TABELLE 2, wobei das Kit Reagenzien umfasst, die für RT-PCR notwendig sind.

13. Verwendung eines Kits in dem Verfahren nach einem der Ansprüche 8 bis 11, wobei das Kit Reagenzien und einen ersten Sondensatz umfasst, umfassend eine erste Sonde, die spezifisch DPT erkennt, und eine zweite Sonde, die spezifisch einen zweiten Marker erkennt, mit Ausnahme von Histon 1 H3d, ausgewählt aus TABELLE 2.

14. Kit nach Anspruch 12 oder Verwendung nach Anspruch 13, zudem umfassend eine Vorrichtung zum Umwandeln des Expressionsniveaus von DPT und des Expressionsniveaus von mindestens einem zusätzlichen Marker, mit Ausnahme von Histon 1 H3d aus TABELLE 2, in einen Indikator der Wahrscheinlichkeit eines Rezidivs von Prostatakrebs.

15. Kit nach Anspruch 12 oder Verwendung nach Anspruch 13, wobei die Vorrichtung ein Nomogramm ist.

16. Kit oder Verwendung nach Anspruch 15, wobei das Nomogramm als ein oder mehrere Mitglieder der Gruppe verwirklicht wird, bestehend aus einer mechanischen Vorrichtung, einer graphischen Darstellung, und Software-Anweisungen, so dass eine Benutzerschnittstelle zum Bereitstellen einer Darstellung des Indikators für die Wahrscheinlichkeit eines Rezidivs von Prostatakrebs verwirklicht wird.

## Revendications

1. Procédé de distinction entre des sujets dans une catégorie à risque de récidive élevé de cancer de la prostate et des sujets dans une catégorie à risque de récidive faible de cancer de la prostate, comprenant :
(i) la mesure d'un taux d'expression d'au moins deux marqueurs diagnostiques choisis parmi l'ensemble de marqueurs, sauf l'histone 1 H3d, présentés dans le TABLEAU 2, chaque taux d'expression étant mesuré par rapport à une moyenne des taux d'expression de deux marqueurs témoins ou plus et
(ii) la combinaison du taux d'expression des au moins deux marqueurs diagnostiques avec une probabilité prédictive basée sur un nomogramme de Kattan, pour assigner aux sujets l'une de la catégorie à risque de récidive élevé de cancer de la prostate et la catégorie à risque de récidive faible de cancer de la prostate.

2. Procédé de la revendication 1, dans lequel le nomogramme de Kattan est une mesure composite basée sur deux membres ou plus d'un groupe constitué des valeur de PSA avant chirurgie (et avant hormonothérapie, le cas échéant), PSA au moment de la prostatectomie, Gleason primaire à la chirurgie, Gleason secondaire à la chirurgie, somme de Gleason de prostatectomie, année de prostatectomie, mois sans maladie, le fait que les marges chirurgicales sont positives ou non, le fait que le cancer est observé ou non dans les vésicules séminales, le fait qu'une extension extra-capsulaire est présente ou non, le fait que le cancer est présent ou non dans les ganglions lymphatiques(si certains ont été enlevés), PSA pré-radiothérapie, dose de rayonnement (le cas échéant), le fait que les marges chirurgicales sont positives ou négatives (le cas échéant), présence ou non d'une atteinte des vésicules séminales (le cas échéant), présence ou non d'une atteinte des ganglions lymphatiques (le cas échéant), présence ou non d'une atteinte extra-capsulaire (le cas échéant), prescription ou non d'hormones néo-adjuvantes, prescription ou non d'un rayonnement néo-adjuvant.

3. Procédé de la revendication 1, dans lequel le nomogramme de Kattan est une mesure composite basée sur deux membres ou plus d'un groupe constitué des taux de PSA pré-traitement, grade de Gleason combiné, somme de Gleason de spécimen, stade clinique, statut de marge chirurgicale, longueur maximale de cancer d'invasion capsulaire prostatique dans une carotte, longueur totale de cancer dans les carottes de biopsie, pourcentage de taux positif de carottes, extension extra-prostatique, taux d'extension extra-prostatique, indice apoptotique, pourcentage de cancer dans une ou plusieurs carottes, pourcentage de cancer de grade élevé dans une ou plusieurs carottes, volume total de tumeur, zone de localisation du cancer, présence d'invasion de vésicules séminales, type d'invasion de vésicule séminale, p53, Ki-67, p27, statut de ploïdie d'ADN, statut des ganglions lymphatiques, et invasion lympho-vasculaire.

4. Procédé de la revendication 1, dans lequel le classificateur comprend des marqueurs diagnostiques MYH11, SSBP1 et DPT.

5. Procédé de la revendication 4, dans lequel le classificateur comprend des marqueurs diagnostiques autres que l'histone 1 H3d, choisis dans le TABLEAU 2, en plus de MYH11, SSBP1 et DPT.

6. Procédé de la revendication 1, dans lequel les marqueurs témoins sont TUBA, ALAS1 et ACTG1.

7. Procédé de la revendication 1, dans lequel les marqueurs témoins sont choisis pour des taux d'expression relativement stationnaires tels que détectés par RT-PCR.

8. Procédé de prédiction de la récidive de cancer de la prostate comprenant :
la détermination d'un taux d'expression de DPT par rapport à un standard ;
la détermination du taux d'expression d'au moins un marqueur additionnel du TABLEAU 2 ; et
la transformation du taux d'expression de DPT et le taux d'expression d'au moins un marqueur additionnel, sauf l'histone 1 H3d, du TABLEAU 2 en score correspondant à une probabilité de récidive de cancer de la prostate.

9. Procédé de la revendication 8 comprenant en outre, en tant qu'alternative à l'étape de transformation de combinaison du taux d'expression de DPT et du taux d'expression d'au moins un marqueur additionnel, sauf l'histone 1 H3d, du TABLEAU 2 avec au moins un indicateur additionnel de récidive de cancer de la prostate pour déterminer un score composite.

10. Procédé de la revendication 9 dans lequel le score composite reflète une probabilité de récidive de cancer de la prostate.

11. Procédé de la revendication 8, dans lequel le taux d'expression de DPT et le taux d'expression d'au moins un marqueur additionnel, sauf l'histone 1 H3d, du TABLEAU 2 est déterminé avant au moins un traitement choisi dans le groupe constitué des prostectomie, hormonothérapie, chimiothérapie à un agent unique, chimiothérapie à deux agents et traitement avec un inhibiteur de farnésyle transférase.

12. Kit de détection d'un taux d'expression d'au moins DPT, le kit comprenant des réactifs et un premier ensemble de sondes comprenant une première sonde reconnaissant spécifiquement DPT et une deuxième sonde reconnaissant spécifiquement un deuxième marqueur, sauf l'histone 1 H3d, choisi dans le TABLEAU 2, le kit comprenant les réactifs nécessaires pour la RT-PCR.

13. Utilisation d'un kit dans le procédé de l'une quelconque des revendications 8 à 11, le kit comprenant des réactifs et un premier ensemble de sondes comprenant une première sonde reconnaissant spécifiquement DPT et une deuxième sonde reconnaissant spécifiquement un deuxième marqueur, sauf l'histone 1 H3d, choisi dans le TABLEAU 2.

14. Kit de la revendication 12 ou utilisation de la revendication 13, comprenant en outre un dispositif pour convertir le taux d'expression de DPT et le taux d'expression d'au moins un marqueur additionnel, sauf l'histone 1 H3d, du TABLEAU 2 en indicateur d'une probabilité de récidive du cancer de la prostate.

15. Kit de la revendication 12 ou utilisation de la revendication 13, le dispositif étant un nomogramme.

16. Kit ou utilisation de la revendication 15 dans lesquels le nomogramme est mis en oeuvre sous la forme d'un ou plusieurs membres du groupe constitué d'un dispositif mécanique, une représentation graphique, et des instructions logicielles pour mettre en oeuvre une interface utilisateur pour fournir une représentation de l'indicateur de la probabilité de récidive du cancer de la prostate.
